# EUROPÄISCHE PATENTSCHRIFT

(11) **EP 3 093 067 B1**
(45) Veröffentlichungstag und Bekanntmachung des Hinweises auf die Patenterteilung: **22.01.2020**
(21) Anmeldenummer: 15180189.1
(22) Anmeldetag: 07.08.2015
(51) Int. Cl.: B01F 13/00, B01F 11/00, B01F 15/02, B01F 13/06, A61B 17/88

(54) **VORRICHTUNG UND VERFAHREN ZUM MISCHEN UND LAGERN VON POLYMETHYMETHACRYLAT-KNOCHENZEMENT**
DEVICE AND METHOD FOR MIXING AND STORING OF POLYMETHYLMETHACRYLATE BONE CEMENT
DISPOSITIF ET PROCÉDÉ DE MELANGE ET DE STOCKAGE DE CIMENT OSSEUX EN POLYMETHACRYLATE DE METHYLE

(30) Priorität: 04.05.2015 DE 102015106899
(43) Veröffentlichungstag der Anmeldung: 16.11.2016
(73) Patentinhaber: Heraeus Medical GmbH, 61273 Wehrheim (DE)
(72) Erfinder: Dr. Kluge, Thomas, 56179 Vallendar (DE); Dr. Vogt, Sebastian, 99092 Erfurt (DE)
(74) Vertreter: Heraeus IP

(56) Entgegenhaltungen:
- WO-A1-2006/058153
- DE-B3-102009 031 178
- DE-T2- 60 218 464
- DE-U1- 20 005 333

## Beschreibung

Die Erfindung betrifft ein Mischsystem zum Mischen von Polymethylmethacrylat-Knochenzement (PMMA-Knochenzement) aus zwei Ausgangskomponenten, insbesondere zum Mischen eines medizinischen Knochenzements, und zur Lagerung der Ausgangskomponenten.

Die Erfindung betrifft ferner ein Verfahren zum Mischen von Polymethylmethacrylat-Knochenzement.

Gegenstand der Erfindung ist somit eine Vorrichtung zur Lagerung und Vermischung von Polymethylmethacrylat-Knochenzement sowie Verfahren zum Mischen von Polymethylmethacrylat-Knochenzement.

Polymethylmethacrylat-(PMMA)-Knochenzemente gehen auf die grundlegenden Arbeiten von Sir Charnley zurück (Charnley, J.: Anchorage of the femoral head prosthesis of the shaft of the femur. J. Bone Joint Surg. 42 (1960) 28-30.). PMMA-Knochenzemente bestehen aus einer flüssigen Monomerkomponente und einer Pulverkomponente. Die Monomerkomponente enthält im Allgemeinen das Monomer Methylmethacrylat und einen darin gelösten Aktivator (N,N-Dimethyl-p-toluidin). Die Pulverkomponente, auch als Knochenzementpulver bezeichnet, weist ein oder mehrere Polymere, einen Röntgenopaker und den Initiator Dibenzoylperoxid auf. Die Polymere der Pulverkomponente werden auf Basis von Methylmethacrylat und Comonomeren, wie Styren, Methylacrylat oder ähnlichen Monomeren durch Polymerisation, vorzugsweise Suspensionspolymerisation, hergestellt. Beim Vermischen der Pulverkomponente mit der Monomerkomponente entsteht durch Quellung der Polymere der Pulverkomponente im Methylmethacrylat ein plastisch verformbarer Teig, der eigentliche Knochenzement. Beim Vermischen der Pulverkomponente mit der Monomerkomponente reagiert der Aktivator N,N-Dimethyl-p-toluidin mit Dibenzoylperoxid unter Bildung von Radikalen. Die gebildeten Radikale initiieren die radikalische Polymerisation des Methylmethacrylats. Mit fortschreitender Polymerisation des Methylmethacrylats erhöht sich die Viskosität des Zementteigs, bis dieser erstarrt.

Das am häufigsten in Polymethylmethacrylat-Knochenzementen verwendete Monomer ist Methylmethacrylat. Redoxinitiatorsysteme bestehen üblicherweise aus Peroxiden, Beschleunigern sowie gegebenenfalls geeigneten Reduktionsmitteln. Eine Radikalbildung erfolgt nur dann, wenn alle Bestandteile der Redoxinitiatorsysteme zusammenwirken. Deshalb werden die Bestandteile des Redoxinitiatorsystems in den separaten Ausgangskomponenten so angeordnet, dass diese keine radikalische Polymerisation auslösen können. Die Ausgangskomponenten sind bei geeigneter Zusammensetzung lagerstabil. Erst bei Vermischung der beiden Ausgangskomponenten zu einem Zementteig reagieren die zuvor getrennt in den beiden Pasten, Flüssigkeiten oder Pulvern gelagerten Bestandteile des Redoxinitiatorsystems, wobei Radikale gebildet werden, welche die radikalische Polymerisation des wenigstens einen Monomers auslösen. Die radikalische Polymerisation führt dann unter Verbrauch des Monomers zu Bildung von Polymeren, wobei der Zementteig aushärtet.

PMMA-Knochenzemente können in geeigneten Mischbechern mit Hilfe von Spateln durch Vermischen des Zementpulvers mit der Monomerflüssigkeit vermischt werden. Nachteilig ist an dieser Vorgehensweise, dass Lufteinschlüsse im gebildeten Zementteig entstehen können beziehungsweise vorhanden sein können, welche die mechanischen Eigenschaften des ausgehärteten Knochenzements negativ beeinflussen können und die somit später eine Destabilisierung des Knochenzements verursachen können.

Zur Vermeidung von Lufteinschlüssen im Knochenzementteig wurden eine Vielzahl von Vakuum-Zementiersystemen vorgeschlagen, von denen exemplarisch folgende genannt sind: US 6 033 105 A, US 5 624 184 A, US 4 671 263 A, US 4 973 168 A, US 5 100 241 A, WO 99/67015 A1, EP 1 020 167 A2, US 5 586 821 A, EP 1 016 452 A2, DE 36 40 279 A1, WO 94/26403 A1, EP 1 005 901 A2, US 5 344 232 A. Bei den dargelegten Vakuum-Zementiersystemen ist es erforderlich, zur Erzeugung des Unterdrucks eine externe Vakuumpumpe anzuschließen. Diese werden im Allgemeinen mit Druckluft unter Nutzung des Venturi-Prinzips betrieben. Die für den Betrieb der Vakuumpumpen notwendige Druckluft wird entweder aus stationären Druckluftanlagen oder auch aus elektrisch betriebenen Kompressoren bezogen. Daneben ist es auch möglich, für die Vakuumerzeugung elektrisch betriebene Vakuumpumpen zu verwenden.

Eine Weiterentwicklung in der Zementiertechnik stellen Zementiersysteme dar, in denen sowohl das Zementpulver als auch die Monomerflüssigkeit bereits in separaten Kompartimenten der Mischsysteme verpackt sind und die erst unmittelbar vor der Zementapplikation im Zementiersystem miteinander vermischt werden. Solche geschlossenen Full-Prepacked-Mischsysteme wurden mit der EP 0 692 229 A1, der DE 10 2009 031 178 B3, der US 5 997 544 A, der US 6 709 149 B1, der DE 698 12 726 T2 und der US 5 588 745 A vorgeschlagen. Auch bei diesen Mischsystemen ist eine externe Vakuumquelle notwendig.

Das Patent DE 10 2009 031 178 B3 offenbart eine gattungsgemäße Mischvorrichtung mit einem zweiteiligen Austragskolben zum Verschluss einer Zementkartusche. Dabei wird eine Kombination aus einem Gas-durchlässigen Sterilisationskolben und einem Gas-undurchlässigen Dichtungskolben verwendet. Dieses Prinzip eines geschlossenen Vakuummischsystems wird beim dem geschlossenen Zementiersystem PALACOS® PRO verwirklicht, das von der Firma Heraeus Medical GmbH hergestellt und vertrieben wird.

In DE 200 05 333 U1 ist eine Aufbereitungs- und Applikationsvorrichtung für Implantatmaterialien beschrieben. Die Vorrichtung enthält einen ersten Behälter zur Aufnahme einer pulver- oder granulatförmigen Komponente und einen Behälter zur Aufnahme einer flüssigen Komponente. Der zweite Behälter enthält Mittel zur Verdrängung der flüssigen Komponente in den ersten Behälter. Der erste Behälter enthält einen hohl ausgebildeten Mischschaft, der an eine Handpumpe zur Erzeugung eines Unterdrucks angeschlossen ist.

Die DE 602 18 464 T2 beschreibt eine Einsetzeinrichtung für Knochentransplantat und offenbart eine Vorrichtung, umfassend: a) einen Auslassanschluss, angepasst zur Befestigung an einer Flüssigkeitsrückhaltekammer, wobei der Anschluss mindestens eine Öffnung besitzt, b) einen ersten und einen zweiten Eingangsanschluss, wobei jeder dieser Anschlüsse angepasst ist zur Befestigung an einer Abgabespritze und wobei jeder dieser Eingangsanschlüsse eine Öffnung aufweist, und c) ein erstes und ein zweites Rohr, wobei jedes dieser Rohre eine sterile Innenfläche und sowohl Eingangs- als auch Ausgangsabschnitte aufweist, d) eine durchgängige Basis mit einer oberen und einer unteren Fläche, wobei jeder Anschluss auf der durchgängigen Basis angebracht ist und sich von der oberen Fläche nach oben erstreckt, der Eingangsabschnitt des ersten Rohrs in Flüssigkeitsverbindung mit der Öffnung des ersten Eingangsanschlusses steht, der Eingangsabschnitt des zweiten Rohrs in Flüssigkeitsverbindung mit der Öffnung des zweiten Eingangsanschlusses steht, der Ausgangsabschnitt jedes Rohres in Flüssigkeitsverbindung mit der mindestens einen Öffnung des Auslassanschlusses steht, wobei jede Anschlussöffnung einen Mittelpunkt hat und wobei die drei Mittelpunkte ein Dreieck beschreiben.

In der WO 00/35506 A1 wird eine Vorrichtung vorgeschlagen, bei der Polymethylmethacrylat-Knochenzementpulver in einer Kartusche gelagert wird, wobei das Zementpulver das gesamte Volumen der Kartusche ausfüllt und die Zwischenräume zwischen den Partikeln des Zementpulvers ein solches Volumen hat, das dem Volumen der Monomerflüssigkeit entspricht, das zur Herstellung von Knochenzementteig mit dem in der Kartusche gelagerten Zementpulver notwendig ist. Diese Vorrichtung ist so aufgebaut, dass durch Vakuumeinwirkung die Monomerflüssigkeit von oben in die Kartusche eingeleitet wird, wobei hierzu ein Vakuum an einem Vakuumanschluss an der Unterseite der Kartusche angelegt wird. Dadurch wird die Monomerflüssigkeit durch das Zementpulver gezogen, wobei die in den Zwischenräumen der Zementpartikel befindliche Luft durch die Monomerflüssigkeit verdrängt wird. Auf eine mechanische Durchmischung des gebildeten Zementteigs mit einem Rührer wird dabei verzichtet.

Nachteilig an diesem System ist, dass Zementpulver, die schnell mit der Monomerflüssigkeit anquellen, mit dieser Vorrichtung nicht angemischt werden können, weil die schnell anquellenden Zementpulverpartikel nach einem Eindringen der Monomerflüssigkeit in das Zementpulver von ungefähr 1 bis 2 cm eine gelartige Barriere bilden und die Migration der Monomerflüssigkeit durch das gesamte Zementpulver behindern. Weiterhin kann bei Vakuumeinwirkung nicht ausgeschlossen werden, dass nach vollständiger Durchdringung des Zementpulvers durch die Monomerflüssigkeit die Monomerflüssigkeit über den Vakuumanschluss abgesaugt wird. Dann steht nicht genügend Monomerflüssigkeit für die Aushärtung durch radikalische Polymerisation zur Verfügung beziehungsweise das Mischungsverhältnis wird ungewollt verändert und damit auch die Konsistenz des Knochenzements. Weiterhin ist es problematisch, dass die zwischen den Zementpulverpartikeln eingeschlossen Luft durch die Monomerflüssigkeit von oben nach unten verdrängt werden soll, weil die gegenüber der Monomerflüssigkeit spezifisch leichtere Luft auf Grund der Schwerkraft das Bestreben hat, im Zementpulver nach oben zu wandern und nicht nach unten in Richtung Vakuumanschluss zu migrieren.

Bei der Verwendung von Vakuummischsystemen zur Zementierung müssen externe Vakuumpumpen beigestellt werden. Diese Vakuumpumpen sind kostenintensiv und müssen nach der Anwendung gereinigt werden. Weiterhin sind Vakuumschläuche zur Verbindung der Vakuumpumpen mit den Vakuummischsystemen notwendig. Diese Vakuumschläuche müssen den Vakuummischsystemen beigelegt sein. Vor dem Mischen mit einem Vakuummischsystem muss daher zuerst die Vakuumpumpe im Operations-Saal (OP-Saal) aufgebaut und an eine Energiequelle, wie Druckluft oder an elektrischen Strom angeschlossen werden. Danach wird die Vakuumpumpe mit einem Vakuumschlauch mit dem Vakuummischsystem verbunden. Diese Montageschritte kosten wertvolle OP-Zeit und sind potentiell fehlerbehaftet. Die Vakuumpumpe und die Verbindungsleitungen zum Vakuummischsystem und zu externen Energiequellen und Versorgungsleitungen benötigen Platz und stellen potentielle Stolperfallen und Hindernisse dar, die den gelegentlich hektischen Ablauf während einer Operation stören können.

Ein interessantes Konzept wird mit der EP 1 886 647 A1 vorgeschlagen. Das Zementpulver ist dabei in einer evakuierten Kartusche gelagert und die Monomerflüssigkeit befindet sich in einem separaten Behälter. Beim Öffnen der unter Unterdruck stehenden Kartusche wird die Monomerflüssigkeit in die Kartusche gesaugt ohne dass Luft einströmt. Es entsteht ein von Lufteinschlüssen freier Knochenzementteig. Dieses Konzept erfordert es, dass die Kartusche während der Lagerung vor ihrer Verwendung vakuumdicht verschlossen bleibt und keine unsterile Luft eindringen kann. Die Kartusche muss dazu stabil hermetisch abgedichtet sein. Nachteilig ist hieran also, dass der Aufbau aufwendig ist und dass der Inhalt der Kartusche nach dem Einsaugen der Monomerflüssigkeit nicht von einem extern zu bedienenden Mischsystem durchmischt werden kann, da eine Durchführung für eine Mischstange oder ein Mischrohr nicht ohne weiteres dauerhaft vakuumdicht ist. Alle bisher bekannten Full-Prepacked-Mischsysteme nutzen Vakuum oder ein Unterdruck, um die Monomerflüssigkeit in das Zementpulver zu überführen.

Die Aufgabe der Erfindung besteht also darin, die Nachteile des Stands der Technik zu überwinden. Insbesondere sollen die Nachteile der bekannten Vakuummischsysteme mit externer Vakuumquelle überwunden werden. Die Erfindung hat dabei unter anderem die Aufgabe, eine einfache, geschlossene Vorrichtung zu entwickeln, bei der Polymethylmethacrylat-Knochenzementpulver (Zementpulver) und Monomerflüssigkeit in separaten Kompartimenten gelagert und anschließend vermischt werden können. Vom medizinischen Anwender soll das Polymethylmethacrylat-Knochenzementpulver mit der Monomerflüssigkeit innerhalb der Vorrichtung zusammengeführt und vermischt werden können, ohne dass beide Zementkomponenten mit dem medizinischen Anwender in Berührung kommen. Ein Kontakt des medizinischen Anwenders mit dem Polymethylmethacrylat-Knochenzementpulver und mit der Monomerflüssigkeit soll möglichst ausgeschlossen sein. Bei der zu entwickelnden Vorrichtung handelt es sich um ein Full-Prepacked-Mischsystem. Die Vorrichtung soll so beschaffen sein, dass ein Transfer der Monomerflüssigkeit in das Polymethylmethacrylat-Knochenzementpulver ohne die Verwendung von durch Druckluft oder Kompressoren angetriebenen externen Vakuumpumpen, transferiert werden kann. Wichtig ist ferner, dass die Vorrichtung ohne externe Energiequellen, wie Druckluft, Vakuum oder elektrischer Strom, auch unter einfachsten äußeren Bedingungen funktionsfähig und zuverlässig die Herstellung von Knochenzementteig gewährleistet. Die Vorrichtung soll ohne zusätzliche technische Ausrüstung autonom verwendungsfähig sein.

Weiterhin hat die Erfindung die Aufgabe, eine Vorrichtung bereit zu stellen, bei der es möglich ist, dass Volumen an Monomerflüssigkeit, die in das Zementpulver transferiert wird, gezielt zu steuern, so dass das Verhältnis des Volumens an Monomerflüssigkeit zur Zementpulvermenge variiert werden kann, um die Konsistenz und damit die Verarbeitungseigenschaften des Knochenzements zu steuern.

Es soll ferner ein Verfahren bereitgestellt werden, das einen Monomertransfer und ein Mischen in Full-Prepacked-Mischsystemen ermöglicht. Dabei soll das zu entwickelnde Mischsystem, hauptsächlich aus kostengünstigem Kunststoff gefertigt werden können.

Des Weiteren soll eine kostengünstig zu fertigende und zuverlässig funktionierende Vorrichtung zum Mischen eines medizinischen Zements und gegebenenfalls zur Lagerung der Ausgangskomponenten des Zements sowie ein Verfahren zum Mischen des Knochenzements gefunden werden, bei dem eine möglichst einfache manuelle Bedienung zum Mischen der Ausgangskomponenten angewendet werden kann, möglichst ohne dass eine externe oder zusätzliche Energiequelle verwendet werden muss und ohne dass Lufteinschlüsse in dem Mischgut entstehen.

Die Hauptkomponente des Polymethylmethacrylat-Knochenzements als Mischgut soll ein Pulver sein und die zweite Komponente soll in Form einer Flüssigkeit vorliegen. Die beiden Ausgangskomponenten des Knochenzements sollen bevorzugt in dem Full-Prepacked-Mischsystem getrennt gelagert werden können und durch Anwendung der Vorrichtung sicher zusammengeführt werden können.

Die Aufgaben der Erfindung werden gelöst durch eine Vorrichtung zum Mischen von Polymethylmethacrylat-Knochenzement (PMMA-Knochenzement) und zum Lagern der Ausgangskomponenten des Knochenzements, insbesondere einer Momonerflüssigkeit und eines Zementpulvers als Ausgangskomponenten des Knochenzements, die Vorrichtung aufweisend
1) eine Kartusche mit einem Innenraum zum Mischen des Knochenzements, der auf einer Seite durch einen beweglichen Austragskolben verschlossen ist,
2) einen Monomer-Behälter für eine Monomerflüssigkeit und/oder einen Anschluss zur Befestigung eines Monomer-Behälters für eine Monomerflüssigkeit, so dass der Monomer-Behälter in der Vorrichtung derart zu öffnen ist, dass die Monomerflüssigkeit aus dem Monomer-Behälter in die Vorrichtung ausläuft,
3) eine Verbindungsleitung, durch die die Monomerflüssigkeit in den Innenraum der Kartusche zu leiten ist, wobei
4) ein Hohlzylinder mit der Verbindungsleitung verbunden ist und der Hohlzylinder zwischen dem Monomer-Behälter oder dem Anschluss für den Monomer-Behälter und dem Innenraum der Kartusche angeordnet ist, wobei in dem Hohlzylinder ein axial im Hohlzylinder verschiebbarer Pumpkolben angeordnet ist, wobei die Monomerflüssigkeit aus dem geöffneten Monomer-Behälter in den Hohlzylinder fließen kann und die Verbindungsleitung den Hohlzylinder mit dem Innenraum der Kartusche derart verbindet, dass mit dem Pumpkolben Monomerflüssigkeit aus dem Hohlzylinder durch Betätigen des Pumpkolbens durch die Verbindungsleitung in den Innenraum der Kartusche zu drücken ist,
wobei der Monomer-Behälter (46, 96) für die Monomerflüssigkeit oder der Anschluss (20, 70) zur Befestigung des Monomer-Behälters (46, 96) an einer Mantelfläche des Hohlzylinders (12, 62) unmittelbar unterhalb des Pumpkolbens (14, 64) in den Hohlzylinder (12, 62) mündet..

Dass der Hohlzylinder zwischen dem Monomer-Behälter für die Monomerflüssigkeit oder dem Anschluss für den Monomer-Behälter und der Kartusche angeordnet ist, bedeutet nicht, dass der Hohlzylinder geometrisch dazwischen angeordnet ist, sondern bezüglich der Fluidverbindungen also der Flussrichtung der Monomerflüssigkeit, wenn diese aus dem geöffneten Monomer-Behälter in Richtung der Kartusche fließt beziehungsweise gepumpt wird, zwischen dem Monomer-Behälter oder dem Anschluss für den Monomer-Behälter und der Kartusche angeordnet ist.

Damit die Monomerflüssigkeit aus dem geöffneten Monomer-Behälter in den Hohlzylinder fließt, ist der geöffnete Monomer-Behälter hierzu mit dem Hohlzylinder verbunden, vorzugsweise über eine Einmündung mit dem Hohlzylinder verbunden.

Damit die Monomerflüssigkeit ohne zusätzliche Krafteinwirkung fließen kann, muss die Vorrichtung bestimmungsgemäß aufgestellt werden, damit die Gravitation die gewünschte Flussrichtung bewirkt. Demzufolge sind die im Rahmen der vorliegenden Erfindung verwendeten Begriffe oben und unten sowie oberhalb und unterhalb und höchsten und tiefsten immer in Bezug auf die bestimmungsgemäße Aufstellung der Vorrichtung bezogen.

Der Innenraum der Kartusche hat vorzugsweise eine zylindrische Geometrie. Die zylindrische Form ist die einfachste, mit der sich der Innenraum der Kartusche und der Hohlzylinder realisieren lassen. Unter einer zylindrischen Form ist geometrisch die Form eines allgemeinen Zylinders mit einer beliebigen Grundfläche zu verstehen, also nicht nur einer mit einer kreisförmigen Grundfläche. Die Innenwand des Innenraums kann also ein Zylinder mit beliebiger Grundfläche sein und der Mantel des Hohlzylinders kann ein Zylinder mit beliebiger Grundfläche sein, das heißt auch mit nicht kreisförmiger oder runder Grundfläche. Erfindungsgemäß wird jedoch eine zylindrische Geometrie mit rotationssymmetrischer Grundfläche bevorzugt.

Um eine gute Pumpwirkung zu erreichen und einen Austritt von Monomerflüssigkeit aus dem Pumpkolben zu vermeiden, schließt der Pumpkolben fluiddicht mit den Innenwänden des Hohlzylinders ab. Hierzu kann eine umlaufende Dichtung vorgesehen sein, die den Pumpkolben mit den Innenwänden des Hohlzylinders abschließt.

Es ist erfindungsgemäß bevorzugt, wenn an den Anschluss für den Monomer-Behälter der Monomer-Behälter angeschlossen oder in den Anschluss für den Monomer-Behälter der Monomer-Behälter eingesetzt ist.

Der Polymethylmethacrylat-Knochenzement wird bevorzugt aus zumindest zwei Komponenten gemischt beziehungsweise ist aus zumindest zwei Komponenten herstellbar. Eine Komponente ist flüssig (die Monomerflüssigkeit) und die andere Komponente ist pulverförmig.

Die Ausgangskomponenten für das Mischgut, insbesondere für den PMMA-Knochenzement, sind erfindungsgemäß bereits in der Kartusche und dem Monomer-Behälter enthalten.

Die Vorrichtung ist erfindungsgemäß bevorzugt auch zum Lagern der Ausgangskomponenten geeignet, insbesondere dann, wenn die Behälter in die Vorrichtung eingesetzt sind oder die Behälter ein fester Teil der Vorrichtung sind.

Es wird erfindungsgemäß vorgeschlagen, dass in der Kartusche eine Mischeinrichtung angeordnet ist, die von außen bedienbar ist, wobei vorzugsweise die Mischeinrichtung über eine Mischstange bedienbar ist, die durch eine Durchführung in dem Austragskolben ins Innere der Kartusche geführt und beweglich gelagert ist.

Besonders bevorzugt ist die Mischstange in der Durchführung drehbar und in Längsrichtung verschiebbar gelagert. Mit der Mischeinrichtung kann der Inhalt des Innenraums der Kartusche bequem mit der Mischstange durchmischt werden. Bei der Verwendung von niedrig-viskosen Knochenzementen kann auf die Verwendung einer Mischstange und einer Mischeinrichtung verzichtet werden, weil die Monomerflüssigkeit vor dem Anquellen des Zementpulvers die Luft der Porenräume zwischen den Zementpulverpartikeln verdrängt hat und die Zementpulverpartikel benetzt hat.

Es kann auch vorgesehen sein, dass der Austragskolben für Pulver undurchlässig ist, wobei bevorzugt in dem Austragskolben ein Porenfilter angeordnet ist, der für Gas durchlässig ist und für Pulver undurchlässig ist.

Der Porenfilter kann bevorzugt als Porenscheibe ausgebildet sein. Durch die Undurchlässigkeit für Pulver kann verhindert werden, dass das Zementpulver aus dem Inneren der Kartusche austreten kann. Wenn der Austragskolben gasdurchlässig ist, kann der Innenraum durch den Austragskolben evakuiert und mit einem Gas, wie beispielsweise Ethylenoxid, sterilisiert werden.

Es wird mit der Erfindung auch vorgeschlagen, dass das Zementpulver im Innenraum der Kartusche enthalten ist.

Es kann auch vorgesehen sein, dass die Monomerflüssigkeit in dem Monomer-Behälter enthalten ist. Hierdurch bildet die Vorrichtung ein fertiges Full-Prepacked-Mischsystem, das nicht vor der Anwendung mit dem Zementpulver gefüllt werden muss. In der Kartusche wird das Zementpulver vor der Verwendung separat zur Monomerflüssigkeit gelagert.

Ferner kann vorgesehen sein, dass zwischen der Verbindungsleitung und dem Innenraum der Kartusche ein für das Zementpulver undurchlässiger und für die Monomerflüssigkeit durchlässiger Filter angeordnet ist.

Hierdurch kann verhindert werden, dass das Zementpulver in die Verbindungsleitung eindringt und dort beim Einleiten der Monomerflüssigkeit polymerisiert und so ungewollt die Verbindungsleitung verstopft beziehungsweise verklebt.

Gemäß einer Weiterbildung der vorliegenden Erfindung kann vorgesehen sein, dass die Vorrichtung einen Standfuß aufweist, in dem zumindest ein Teil der Verbindungsleitung angeordnet ist, wobei die Kartusche lösbar mit dem Standfuß verbunden ist, insbesondere über ein Schraubgewinde lösbar mit dem Standfuß verbunden ist, wobei bevorzugt gegebenenfalls der für das Zementpulver undurchlässige und für die Monomerflüssigkeit durchlässige Filter in dem Standfuß der Vorrichtung angeordnet ist, besonders bevorzugt in der Verbindung zur Kartusche des Standfußes angeordnet ist.

Hierdurch kann die Vorrichtung leicht aufgestellt und einfach bedient werden.

Dabei kann vorgesehen sein, dass der Hohlzylinder und der Monomer-Behälter oder der Hohlzylinder und der Anschluss zur Befestigung des Monomer-Behälters mit dem Standfuß verbunden sind, bevorzugt unlösbar mit dem Standfuß verbunden sind.

Hierdurch wird ein besonders einfacher und kostengünstiger Aufbau der Vorrichtung ermöglicht.

Es ist vorgesehen, dass der Monomer-Behälter für die Monomerflüssigkeit oder der Anschluss zur Befestigung des Monomer-Behälters an einer Mantelfläche des Hohlzylinders unmittelbar unterhalb des Pumpkolbens in den Hohlzylinder mündet.

Hierdurch kann sichergestellt werden, dass die Monomerflüssigkeit vollständig in den Hohlzylinder fließen kann und den Hohlzylinder füllen kann. Zudem kann so Luft an dieser Stelle besonders leicht aus dem Hohlzylinder austreten.

Des Weiteren kann vorgesehen sein, dass die Vorrichtung ein Öffnungsmittel zum Öffnen des Monomer-Behälters aufweist, mit dem der Monomer-Behälter innerhalb der Vorrichtung zu öffnen ist, wobei bevorzugt in der Verbindung zum Hohlzylinder ein Sieb oder ein Filter angeordnet ist, mit dem Bruchstücke oder Fetzen des geöffneten Monomer-Behälters zurückgehalten werden können.

Bevorzugt kann dabei vorgesehen sein, dass der Monomer-Behälter eine aufbrechbare Glasampulle ist.

Durch das Öffnungsmittel als Teil der Vorrichtung kann die Vorrichtung auch langfristig zum Lagern des Monomers verwendet werden. Eine geeignetes Öffnungsmittel ist beispielsweise aus dem Patent DE 10 2010 026 496 B4 bekannt.

Mit einer Weiterbildung der Erfindung zur Ausnutzung der Gravitation als Antrieb für den Fluss der Monomerflüssigkeit in den Hohlzylinder wird vorgeschlagen, dass der Monomer-Behälter oberhalb der der Verbindung zum Hohlzylinder angeordnet ist.

Dadurch kann die Monomerflüssigkeit aus dem Monomer-Behälter nach dem Öffnen des Monomer-Behälters aufgrund der Gravitation in den Hohlzylinder fließen. Alternativ könnte der Monomer-Behälter auch ausgepresst werden und so in den Hohlzylinder fließen.

Bevorzugte Vorrichtungen können auch dadurch auszeichnen, dass die Verbindungsleitung an der unteren Seite mit dem Hohlzylinder verbunden ist, bevorzugt am tiefsten Punkt des Hohlzylinders mit dem Hohlzylinder verbunden ist, wobei besonders bevorzugt der Pumpkolben auf der gegenüberliegenden Seite des Hohlzylinders angeordnet ist.

Hierdurch kann die Monomerflüssigkeit mit dem Pumpkolben vollständig aus dem Hohlzylinder abfließen beziehungsweise herausgedrückt werden.

Es wird ferner vorgeschlagen, dass der Hohlzylinder auf der dem Pumpkolben gegenüberliegenden Seite einen kegelförmigen, halbkugelförmigen oder anderen sich nach unten verjüngenden Boden aufweist, wobei bevorzugt die dem Boden des Hohlzylinders zugewandte Fläche des Pumpkolbens eine Negativform des Bodens bildet.

Hierdurch kann die Monomerflüssigkeit mit dem Pumpkolben vollständig aus dem Hohlzylinder abfließen beziehungsweise herausgedrückt werden. Das bedeutet, dass die gesamte Monomerflüssigkeit zum tiefsten Punkt des Hohlzylinders fließt und keine "toten" Bereiche vorhanden sind, in denen Monomerflüssigkeit bei Betätigung des Pumpkolbens zurück bleibt. Durch die Anpassung der Form des Pumpkolbens an die innere Form des Hohlzylinders wird die gesamte Monomerflüssigkeit aus dem Hohlzylinder durch den Pumpkolben bei Bewegung des Pumpkolbens in Richtung der Öffnung zur Verbindungsleitung gepresst, ohne dass Rückstände der Monomerflüssigkeit im Hohlzylinder verbleiben. Weiterhin wird durch diese kegelförmige oder kugelförmige beziehungsweise passende Gestalt der Stirnseite des Pumpkolbens gewährleistet, dass bei Bewegung des Pumpkolbens nach unten beziehungsweise in Richtung des Standfußes, die Luft über der Monomerflüssigkeit im Hohlzylinder durch die Öffnung in der Mantelfläche des Hohlzylinders entweichen kann und keine Luftblasen oberhalb der Monomerflüssigkeit beim Transferieren der Monomerflüssigkeit in den Innenraum der Kartusche beziehungsweise in das Zementpulver verbleiben.

Bevorzugte Ausführungsformen können vorsehen, dass der Pumpkolben manuell axial im Hohlzylinder bewegt werden kann, bevorzugt manuell axial in den Hohlzylinder eingepresst werden kann.

Damit ist es möglich, die Monomer-Flüssigkeit manuell aus dem Hohlzylinder auszupressen und in den Innenraum der Kartusche zu überführen.

Zur Vereinfachung der Bedienung und für eine höhere Variabilität erfindungsgemäßer Vorrichtungen kann auch vorgesehen sein, dass der Hohlzylinder transparent ist und Markierungen zur Füllstandsmenge einer Flüssigkeit in dem Hohlzylinder aufweist.

Dadurch kann eine durch die Markierungen bestimmte Menge der Monomerflüssigkeit in den Hohlzylinder gefüllt werden beziehungsweise aus dem Hohlzylinder in den Innenraum der Kartusche eingepresst werden. So ergibt sich die Möglichkeit mit der Vorrichtung einen Knochenzementteig mit einer durch die Menge der Monomerflüssigkeit vorgegebenen Konsistenz herzustellen. Alternativ kann der Hohlzylinder auch nicht transparent sein und Markierungen an dem aus dem Hohlzylinder ragenden Ende des Pumpkolbens vorgesehen sein, um einen definierten Vortrieb des Pumpkolbens zu ermöglichen und damit ein definiertes Volumen der Monomerflüssigkeit aus dem Hohlzylinder pressen zu können. Mit derartigen Aufbauten ist es also möglich, sowohl das gesamte Volumen der Monomerflüssigkeit aus dem Hohlzylinder in das Zementpulver im Innenraum der Kartusche zu pressen als auch nur bestimmte Teilvolumen der Monomerflüssigkeit aus dem Hohlzylinder in das Zementpulver zu transferieren. Dadurch kann das Verhältnis von Monomerflüssigkeit zur Pulvermenge eingestellt werden, wodurch das zeitliche Erreichen der Klebfreiheit des gebildeten Zementteigs und die Viskosität des Knochenzements gezielt gesteuert werden kann.

Es kann auch vorgesehen sein, dass der Hohlzylinder ein Innengewinde aufweist und der Pumpkolben ein dazu passendes Außengewinde aufweist, so dass der Pumpkolben in den Hohlzylinder einschraubbar ist, um die Monomerflüssigkeit aus dem Hohlzylinder in den Innenraum der Kartusche zu pressen.

Auch hierdurch kann eine definierte Menge der Monomerflüssigkeit aus dem Hohlzylinder in den Innenraum der Kartusche eingepresst werden. Dadurch ergibt sich die Möglichkeit mit der Vorrichtung einen Knochenzementteig mit einer durch die Menge der Monomerflüssigkeit bestimmten Konsistenz herzustellen.

Alternativ kann vorgesehen sein, dass die Vorrichtung eine gespannte Druckfeder und eine Arretierung aufweist, wobei die Druckfeder und/oder der Pumpkolben mit der Arretierung lösbar arretiert ist oder sind, wobei bei gelöster Arretierung die Druckfeder einen Druck auf den Pumpkolben ausübt, so dass der Pumpkolben in den Hohlzylinder gepresst wird.

Für die Ausführung, bei der der Hohlzylinder ein Innengewinde und der Pumpkolben ein Außengewinde aufweist, kann analog hierzu eine gespannte Schneckenfeder vorgesehen sein, die nach Lösen einer Arretierung den Pumpkolben in den Hohlzylinder schraubt.

Diese Maßnahmen haben den Vorteil, dass die Bedienung der Vorrichtung vereinfacht wird. Zudem können so mögliche Fehlbedienungen verhindert werden.

Ferner kann vorgesehen sein, dass der Austragskolben mit einer lösbaren Rastvorrichtung mit der Kartusche verbunden ist, wobei die Rastvorrichtung manuell, insbesondere durch axiale Krafteinwirkung lösbar ist, so dass der Austragskolben axial im Innenraum der Kartusche bewegt werden kann.

Hierdurch kann eine ungewollte Bewegung des Austragskolbens, wie sie beispielsweise durch ein Vakuum im Innenraum der Kartusche verursacht werden kann, verhindert werden.

Zur Vermeidung einer ungewollten Befüllung des Innenraums der Kartusche mit Monomerflüssigkeit kann vorgesehen sein, dass die Verbindungsleitung zwischen dem Hohlzylinder und dem Innenraum der Kartusche eine nach oben weisende Schlaufe aufweist, wobei der höchste Punkt der Schlaufe oberhalb der Einmündung des Monomer-Behälters oder des Anschlusses für den Monomer-Behälter in den Hohlzylinder liegt.

Damit kann verhindert werden, dass die Monomerflüssigkeit beim Einfüllen in den Hohlzylinder bereits durch die Verbindungsleitung in den Innenraum der Kartusche gelangt. Diese umgekehrt U-förmige Schlaufe der Verbindungsleitung erreicht, dass vor Bewegung des Pumpkolbens in Richtung der Verbindungsleitung zur Kartusche die Monomerflüssigkeit im Hohlzylinder bis zur Höhe des Scheitelpunkts in der Verbindungsleitung verbleibt, wodurch ein vorzeitiger Eintritt der Monomerflüssigkeit zum Zementpulver verhindert wird. Insbesondere bei hoch-viskosen Zementen kann ein vorzeitiger Kontakt von schon geringen Volumina der Monomerflüssigkeit mit dem Zementpulver zur Verklebung der Verbindungsleitung oder eines als Düse ausgebildeten Leitungsmittels, wie in US 8 662 736 B2 beschrieben, führen. Die Verbindungsleitung kann transparent oder transluzent sein, damit der Anwender den Monomertransfer visuell kontrollieren kann. Hierzu kann insbesondere ein Sichtfenster in der Vorrichtung vorgesehen sein, durch das die Schlaufe mit dem höchsten Scheitelpunkt zu erkennen ist.

Des Weiteren kann erfindungsgemäß vorgesehen sein, dass das Volumen im Hohlzylinder kleiner oder gleich dem Volumen der Monomerflüssigkeit in dem Monomer-Behälter ist.

Dadurch wird verhindert, dass bei Betätigung des Pumpkolbens Luft mit in das Zementpulver gepresst werden kann.

Ferner kann vorgesehen sein, dass der Innenraum der Kartusche an der unteren Seite mit der Verbindungsleitung flüssigkeitsdurchlässig verbunden ist.

Die Verbindungsleitung kann in der Stirnseite des Innenraums in eine Düse gemäß US 8 662 736 B4 münden. Diese Düse verhindert einen Zutritt von Zementpulver in das Leitungsmittel.

In einer anderen Ausgestaltungsvariante ist der Innenraum der Kartusche an einer seitlichen Mantelfläche mit der Verbindungsleitung flüssigkeitsdurchlässig verbunden. Es ist damit auch möglich, die Monomerflüssigkeit seitlich in das Zementpulver in den Innenraum der Kartusche zu transferieren.

Bevorzugt bestehen der Hohlzylinder, die Kartusche und die Verbindungsleitung sowie, sofern vorhanden, auch der Standfuß, die Mischeinrichtung und die Mischstange aus einem Kunststoff und können durch Kunststoffspritzgießen kostengünstig hergestellt werden.

Die der vorliegenden Erfindung zugrundeliegenden Aufgaben werden auch gelöst durch ein Verfahren zum Mischen eines Knochenzements, insbesondere mit einer erfindungsgemäßen Vorrichtung, mit den chronologischen Schritten:
A) ein Monomer-Behälter wird geöffnet,
B) eine Monomerflüssigkeit fließt aus dem Monomer-Behälter in einen Hohlzylinder, wobei der Hohlzylinder auf einer Seite durch einen Pumpkolben begrenzt ist,
C) der Pumpkolben wird in den Hohlzylinder eingedrückt und die Monomerflüssigkeit dadurch aus dem Hohlzylinder und durch eine Verbindungsleitung in den Innenraum einer Kartusche eingepresst, wobei sich in dem Innenraum der Kartusche ein Zementpulver befindet, und
D) die Monomerflüssigkeit und das Zementpulver werden in dem Innenraum der Kartusche gemischt.

Bevorzugt fließt die Monomerflüssigkeit durch Einwirkung der Schwerkraft in den Hohlzylinder.

Dabei kann vorgesehen sein, dass die Monomerflüssigkeit und das Zementpulver erst dann in dem Innenraum der Kartusche gemischt werden, wenn der Pumpkolben vollständig oder bis zu einer Markierung in den Hohlzylinder eingedrückt wurde, wobei die Markierung ein Maß für die in den Innenraum der Kartusche eingeleitete Monomerflüssigkeit ist.

Hierdurch kann sichergestellt werden, dass der erzeugte Knochenzementteig die gewünschte Konsistenz durch die gewünschte Beimengung von Monomerflüssigkeit erhält.

Es wird auch vorgeschlagen, dass die Monomerflüssigkeit und das Zementpulver in dem Innenraum mit einer Mischeinrichtung gemischt werden, indem die Mischeinrichtung durch Bewegen einer drehbar und in Längsrichtung verschiebbar in den Innenraum der Kartusche geführten Mischstange bedient wird, wobei bevorzugt nach dem Mischen die Mischstange bis zum Anschlag aus dem Innenraum der Kartusche herausgezogen wird und besonders bevorzugt die Mischstange nach dem Herausziehen bis zum Anschlag an einer Sollbruchstelle abgebrochen wird.

Hierdurch kann das Verfahren einfach durch manuelle Bedienung durchgeführt werden.

Ferner kann vorgesehen sein, dass der Monomer-Behälter geöffnet wird, in dem ein Öffnungsmittel bedient oder ausgelöst wird, wobei bevorzugt der Monomer-Behälter durch das Öffnungsmittel aufgebrochen wird.

Hierdurch kann der Monomer-Behälter in der Vorrichtung geöffnet werden, so dass die gesamte Vorrichtung nach außen abgeschlossen ist.

Es wird des Weiteren vorgeschlagen, dass der Pumpkolben mit einem gespannten elastischen Federelement in den Hohlzylinder eingedrückt wird, wobei hierfür vorzugsweise zuvor eine Arretierung gelöst wird, die in den Pumpkolben und/oder das Federelement greift.

Damit wird eine weitere Automatisierung des erfindungsgemäßen Verfahrens erreicht und zudem mögliche Fehlbedienungen verhindert.

Schließlich kann auch vorgesehen sein, dass die Kartusche mit dem fertig gemischten Zementteig von der Verbindungsleitung, dem Hohlzylinder und dem Monomer-Behälter gelöst wird und der fertig gemischte Zementteig durch Vortreiben eines Austragskolbens, der axial beweglich in der Kartusche gelagert ist und der den Innenraum der Kartusche auf einer Seite begrenzt, aus dem Innenraum der Kartusche ausgetragen wird.

Der Erfindung liegt die überraschende Erkenntnis zugrunde, dass eine Monomerflüssigkeit mit Hilfe eines Pumpkolbens von unten in den Innenraum einer Kartusche eingepresst werden kann, ohne dass sich hierbei störende Lufteinschlüsse im Knochenzement bilden. Dadurch kann mit der Vorrichtung weitgehend auf externe Energiequellen oder interne Energiespeicher verzichtet werden. Insbesondere müssen keine Vakuumquellen und vakuumdichten Anschlüsse und Bauteile verwendet werden, was den Einsatz an weniger entwickelten Orten sowie im Einsatz vor Ort oder in Feldlazaretten erheblich erleichtert. Zudem sind erfindungsgemäße Full-Prepacked-Mischsysteme unanfälliger für mögliche Störungen und dadurch mit sehr hoher Wahrscheinlichkeit einsatzbereit, weil keine Vakuumlecks auftreten können.

Beispielsweise kann bei einer erfindungsgemäßen Vorrichtung beziehungsweise einem erfindungsgemäßen Verfahren vorgesehen sein, dass nach Öffnung eines Monomer-Behälters die Monomerflüssigkeit durch Schwerkrafteinwirkung in einen Hohlzylinder fließt, aus diesem durch manuelle Betätigung des Pumpkolbens in den Innenraum einer Kartusche gepresst wird, die Zementpulver enthält. Das bedeutet, dass im Gegensatz zu den bisher marktüblichen Mischsystemen der Transfer der Monomerflüssigkeit nicht durch Vakuum sondern durch Druckeinwirkung erfolgt. Ein solcher manuell zu betätigender Monomertransfer durch Druckeinwirkung kann kostengünstig mit einfachen durch Kunststoffspritzgießen herzustellenden Kunststoffteilen realisiert werden. Der besondere Vorteil der erfindungsgemäßen Vorrichtung besteht darin, dass die Vorrichtung ohne äußere Hilfsmittel, wie durch Druckluft angetriebene Vakuumpumpen, und ohne externe Energiequellen, wie Druckluft oder Strom, betrieben werden kann. Die erfindungsgemäße Vorrichtung ist dadurch autonom verwendbar und kann selbst unter einfachsten Operationsbedingungen verwendet werden. Mit der erfindungsgemäßen Vorrichtung wird ein geschlossenes Full-Prepacked-Mischsystem für preissensitive Märkte zur Verfügung gestellt.

Es wurde im Rahmen der vorliegenden Erfindung gefunden, dass bei der durch Druck verursachten Einleitung der Monomerflüssigkeit in das Zementpulver von der Unterseite des Innenraums der Kartusche die Monomerflüssigkeit in Form einer einheitlichen Front von unten nach oben wandert. Dadurch wird die Luft, die sich in den Zwischenräumen zwischen den Zementpulverpartikeln befindet, verdrängt und nach oben heraus gedrückt. Lufteinschlüsse werden dadurch vermieden. Es wurde im Rahmen der vorliegenden Erfindung überraschend gefunden, dass ein mit einer erfindungsgemäßen Vorrichtung und einem erfindungsgemäßen Verfahren hergestellter Knochenzementteig sehr weitgehend frei von Lufteinschlüssen ist und in der Qualität einem unter Vakuum gemischten Zementteig entspricht.

Im Folgenden werden weitere Ausführungsbeispiele der Erfindung anhand von sieben schematisch dargestellten Figuren erläutert, ohne jedoch dabei die Erfindung zu beschränken. Dabei zeigt:
- Figur 1:: eine schematische perspektivische Ansicht einer erfindungsgemäßen Vorrichtung als Full-Prepacked-Mischsystem;
- Figur 2:: die Vorrichtung nach Figur 1 in einer Aufsicht mit zwei Schnittebenen A und B;
- Figur 3:: die Vorrichtung nach den Figuren 1 und 2 in einer schematischen Querschnittansicht entsprechend der Schnittebene A nach Figur 2;
- Figur 4:: einen Teilbereich der Vorrichtung nach den Figuren 1, 2 und 3 in einer schematischen Querschnittansicht entsprechend der Schnittebene B nach Figur 2;
- Figur 5:: eine schematische perspektivische Ansicht einer alternativen erfindungsgemäßen Vorrichtung als Full-Prepacked-Mischsystem;
- Figur 6:: die Vorrichtung nach Figur 5 in einer schematischen Querschnittansicht; und
- Figur 7:: eine schematische Querschnittansicht eines Teilbereichs der Vorrichtung nach den Figuren 4 und 5 und zwar des Hohlzylinders mit einer Spange als Arretierung für eine Druckfeder.

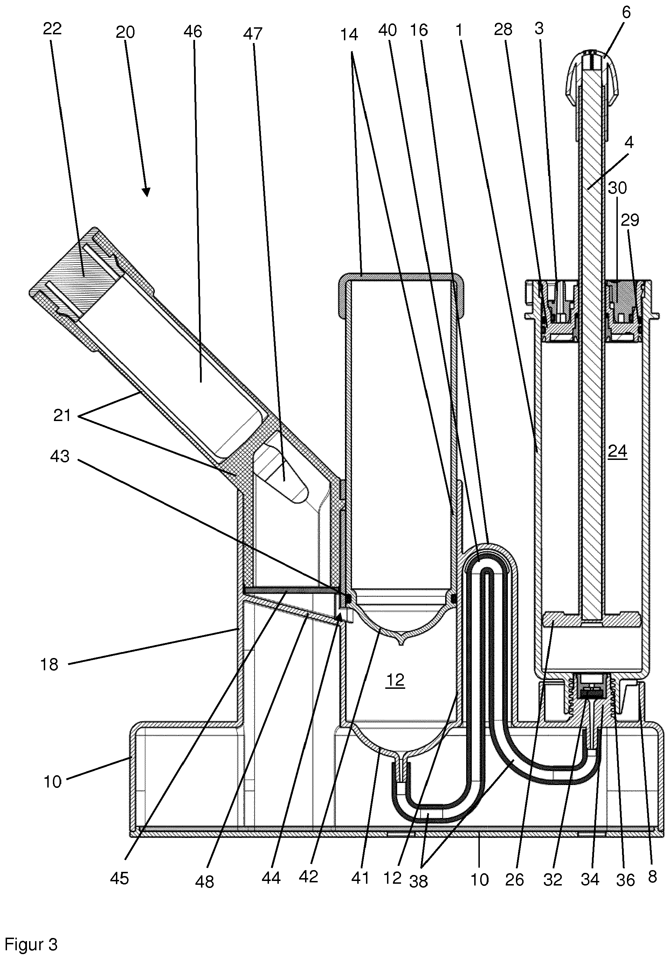

In den Querschnittansichten der Figuren 3, 4, 6 und 7 sind geschnittene Flächen durch Schraffierungen kenntlich gemacht.

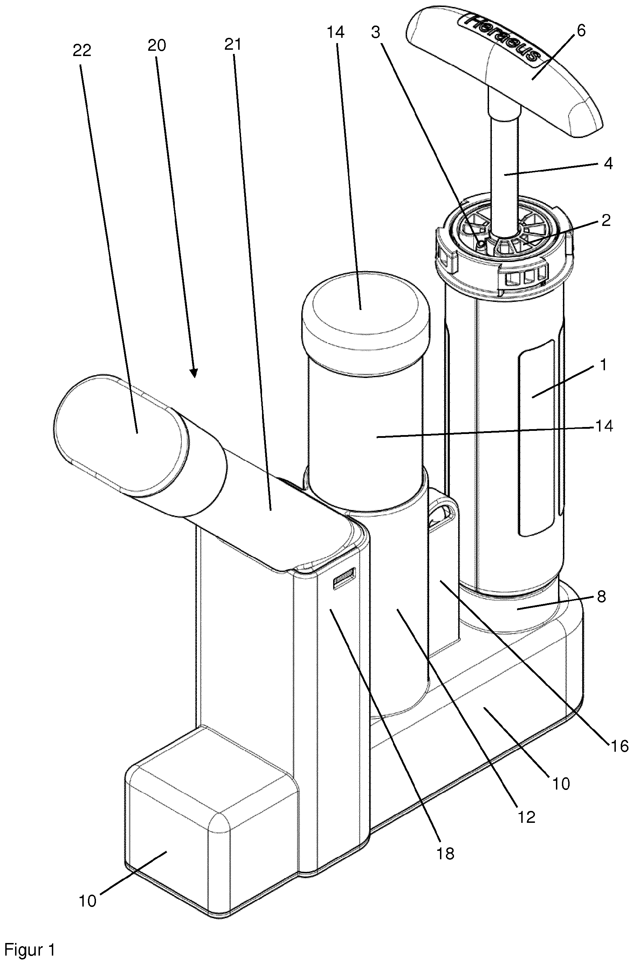

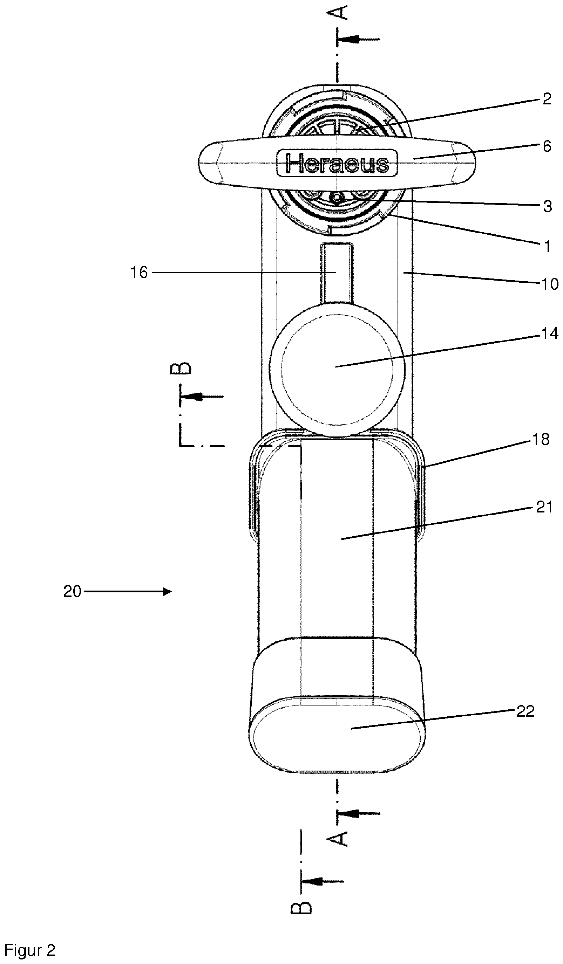

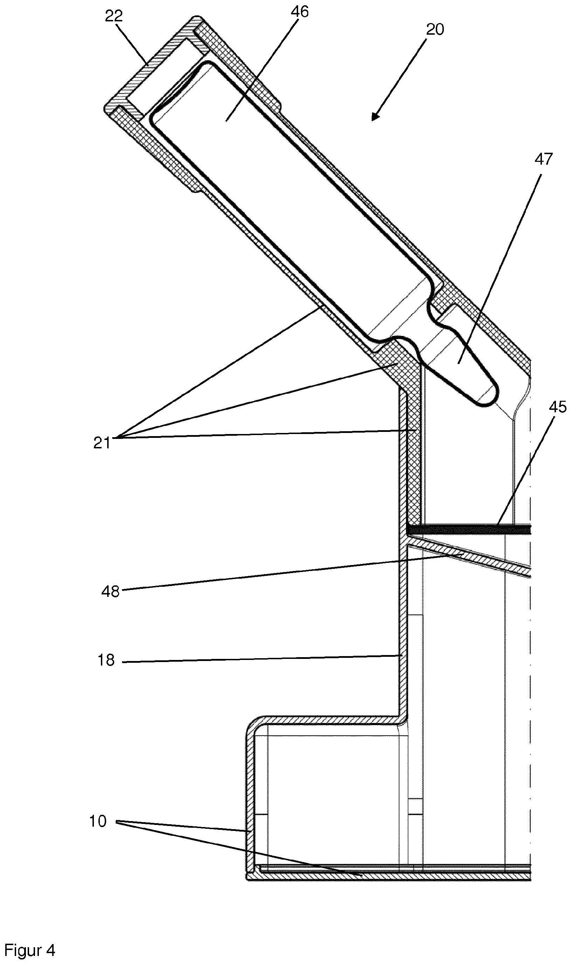

Die Figuren 1 bis 4 zeigen schematische Darstellungen einer ersten erfindungsgemäßen Vorrichtung, die zum Durchführen eines erfindungsgemäßen Verfahrens geeignet ist. Dabei ist in Figur 1 eine schematische perspektivische Ansicht einer erfindungsgemäßen Vorrichtung als Full-Prepacked-Mischsystem dargestellt, in Figur 2 die Vorrichtung nach Figur 1 in einer Aufsicht mit zwei Schnittebenen A und B, in Figur 3 die Vorrichtung nach den Figuren 1 und 2 in einer schematischen Querschnittansicht entsprechend der Schnittebene A nach Figur 2 und in Figur 4 ein Teilbereich dieser Vorrichtung nach den Figuren 1, 2 und 3 in einer schematischen Querschnittansicht entsprechend der Schnittebene B nach Figur 2 dargestellt.

Die Vorrichtung umfasst eine Kartusche 1 in der ein Zementpulver als Ausgangskomponente des herzustellenden PMMA-Knochenzements enthalten ist. Auf der Oberseite (in den Figuren 1 und 3 oben, in Figur 2 in der Richtung des Betrachters) ist die Kartusche 1 mit einem Austragskolben 2 verschlossen. Der Austragskolben 2 kann mit den Wandungen der Kartusche 1 arretiert sein. In dem Austragskolben 2 ist ein Vakuumanschluss 3 vorgesehen, mit dem das Innere der Kartusche 1 evakuiert werden kann und durch den theoretisch auch Ethylenoxid zur Sterilisation des Inhalts der Kartusche 1 eingefüllt werden kann. Durch eine zentrische Durchführung in dem Austragskolben 2 ist eine Mischstange 4 geführt, die sich in die Kartusche 1 hinein und herausziehen lässt und die in dem Austragskolben 2 und damit in der Kartusche 1 drehbar gelagert ist. An dem oberen Ende der Mischstange 4 (in den Figuren 1 und 3 oben, in Figur 2 in der Richtung des Betrachters) ist ein Griffstück 6 zur manuellen Bedienung der Mischstange 4 befestigt.

Im Bereich eines Stutzens 8 an einem Standfuß 10 der Vorrichtung ist die Kartusche 1 mit dem Standfuß 10 lösbar befestigt. Einteilig mit dem Standfuß 10 ausgeführt ist an dessen Oberseite ein Hohlzylinder 12 angeordnet, in dem ein innen passender Pumpkolben 14 angeordnet ist, der sich in das Innere des Hohlzylinders 12 einpressen beziehungsweise eindrücken lässt (in den Figuren 1 und 3 nach unten, in Figur 2 aus Richtung des Betrachters in die Bildebene hinein).

Neben dem Hohlzylinder 12 ist am Standfuß 10 noch ein Anschlussstutzen 18 vorgesehen, der ein Teil eines Anschlusses 20 für einen Monomer-Behälter 46 der Vorrichtung bildet. Der Anschluss 20 für den Monomer-Behälter 46 umfasst neben dem Anschlussstutzen 18 noch eine elastische Aufnahme 21 für den Monomer-Behälter 46 und einen Deckel 22, mit dem der Anschluss 20 für den Monomer-Behälter 46 nach außen abschließbar ist, nachdem der Monomer-Behälter 46 einsteckt wurde. Der Monomer-Behälter 46 ist im Inneren des Anschlusses 20 und in der Vorrichtung angeordnet. Der Monomer-Behälter 46 ist eine Glasampulle, die mit einer Monomerflüssigkeit als zweiter Komponente für den herzustellenden PMMA-Knochenzement gefüllt ist. Die elastische Aufnahme 21 besteht aus Gummi oder einem anderen elastischen Kunststoff.

Weitere Details zum Aufbau der Vorrichtung sind in den Figuren 3 und 4 zu erkennen. Das Innere der Kartusche 1 wird durch einen zylindrischen Innenraum 24 gebildet, in dem das Zementpulver enthalten ist. Zudem ist im Innenraum 24 der Kartusche 1 eine Mischeinrichtung 26 bestehend aus mehreren Mischflügeln 26 angeordnet, die an der Mischstange 4 befestigt ist und die über die Mischstange 4 im Innenraum 24 bewegt werden kann. Der Austragskolben 2 ist zweiteilig aufgebaut und besteht aus einem Sterilisationskolben 28 (in Figur 3 oberer Teil des Austragskolbens) und einem mit einer Dichtung 29 gegen die Innenwand der Innenraums 24 abgedichteten Dichtungskolben 30 (in Figur 3 unterer Teil des Austragskolbens). Der Dichtungskolben 30 weist eine gasdurchlässige aber für Pulver undurchlässige Porenscheibe auf, durch die der Innenraum 24 evakuierbar ist. Der Austragskolben 2 hat einen zylindrischen Außenumfang und schließt dicht mit den Wänden des Innenraums 24 ab. Der Austragskolben 2 kann im Innenraum 24 in Richtung einer Austragsöffnung vorgetrieben werden, die auf der dem Austragskolben 2 gegenüberliegenden Seite des Innenraums 24 der Kartusche 1 angeordnet ist.

Zum Anschließen der Kartusche 1 an den Standfuß 10 ist ein Standfuß-Anschluss 34 mit einem Außengewinde als Verbindung zur Kartusche 1 vorgesehen, in dem ein pulverundurchlässiger und flüssigkeitsdurchlässiger Filter 32 angeordnet ist. Auf der dem Austragskolben 2 gegenüberliegenden Seite des Innenraums 24 der Kartusche 1 ist ein zum Außengewinde des Standfuß-Anschlusses 34 passender Kartuschenanschluss 36 mit Innengewinde vorgesehen. Der Kartuschenanschluss 36 begrenzt die Austragsöffnung der Kartusche 1. Der Kartuschenanschluss 36 ist auf den Standfuß-Anschluss 34 aufgeschraubt und schließt mit diesem dicht ab.

Zwischen dem Hohlzylinder 12 und dem Innenraum 24 der Kartusche 1 ist eine Verbindungsleitung 38 vorgesehen, die den Hohlzylinder 12 mit dem Innenraum 24 der Kartusche 1 verbindet. An der Einmündung in den Innenraum 24 der Kartusche 1 ist der Filter 32 angeordnet, der verhindert, dass Zementpulver aus dem Innenraum 24 der Kartusche 1 in die Verbindungsleitung 38 eindringt. Die Verbindungsleitung 38 bildet eine Schlaufe 40 mit einem hohen Scheitelpunkt, um ein unkontrolliertes Durchfließen der Monomerflüssigkeit durch die Verbindungsleitung 38 in den Innenraum 24 der Kartusche 1 zu verhindern. Ein Gehäuse 16 mit einem kleinen Sichtfenster ist zur Abdeckung und visuellen Kontrolle der Schlaufe 40 vorgesehen.

Der Stutzen 8, Standfuß 10, der Hohlzylinder 12, das Gehäuse 16 und der Anschlussstutzen 18 für den Monomer-Behälter 46 sind einteilig aus einem Kunststoff beispielsweise durch Spritzgießen hergestellt. Der Hohlzylinder 12 ist an der Unterseite (in Figur 3 unten) durch einen trichterförmigen Boden 41 begrenzt, der sich nach unten stetig verjüngt. Hierdurch wird sichergestellt, dass die Monomerflüssigkeit vollständig aus dem Hohlzylinder 12 abfließen beziehungsweise herausgedrückt werden kann. Der Pumpkolben 14 ist durch einen Hohlkörper aus einem Kunststoff gebildet und an der unteren Fläche 42 als Negativ des Bodens 41 des Hohlzylinders 12 geformt. Der Pumpkolben 14 ist mit einer Dichtung 43 gegen die Innenwand des Hohlzylinders 12 abgedichtet und in Längsrichtung (in Figur 3 nach unten) beweglich im Hohlzylinder 12 gelagert.

An einer Mantelfläche des Hohlzylinders 12, direkt unterhalb der unteren Fläche 42 des Pumpkolbens 14, ist eine Einmündung 44 von dem Anschluss 20 für den Monomer-Behälter 46 in den Hohlzylinder 12 vorgesehen. Die Einmündung 44 bildet ein Leitungsmittel für die Monomerflüssigkeit, so dass die Einmündung 44 als Teil einer Verbindungsleitung 38, 44 für die Monomerflüssigkeit aufgefasst werden kann, in der der Hohlzylinder 12 angeordnet ist.

In dem Anschluss 20 für den Monomer-Behälter 46 ist ein Sieb 45 oder Filter 45 angeordnet, mit dem Bruchstücke und Teile des geöffneten Monomer-Behälters 46 abgefangen werden können. Der Monomer-Behälter 46 ist eine Glasampulle 46 mit einem abbrechbaren Ampullenkopf 47 und einem aufbrechbaren Genick, wobei das Genick den Ampullenkopf 47 mit dem Körper der Ampulle 46 verbindet. Durch die Elastizität der Aufnahme 21 für den Monomer-Behälter 46 und durch die Verdickung der Aufnahme 21 im Bereich des Genicks kann durch Biegen der Aufnahme 21 mit dem Monomer-Behälter 46 darin der Kopf 47 des Monomer-Behälters 46 abgebrochen werden. Die Aufnahme 21 mit der entsprechenden Form, insbesondere mit der Verdickung im Bereich des Genicks, bildet dadurch ein Öffnungsmittel 21 zum Öffnen des Monomer-Behälters 46. Andere Öffnungsmittel zum Abscheren des Kopfs 47 des Monomer-Behälters 46 sind ebenfalls umsetzbar.

Im Anschlussstutzen 18 ist unterhalb des Siebs 45 beziehungsweise des Filters 45 eine geneigte Bodenfläche 48 vorgesehen, die in Richtung der Einmündung 44 geneigt ist. Dadurch kann die gesamte Monomerflüssigkeit aus dem Monomer-Behälter 46 durch die Einmündung 44 in den Hohlzylinder 12 fließen.

Ein erfindungsgemäßes Verfahren kann mit der Vorrichtung nach den Figuren 1 bis 4 beispielsweise wie folgt umgesetzt werden. Die Vorrichtung wird mit dem Standfuß 10 auf einem Tisch oder einer anderen passenden ebenen Unterlage aufgestellt. Der Monomer-Behälter 46 wird durch Knicken der elastischen Aufnahme 21 geöffnet, indem der Kopf 47 abgebrochen beziehungsweise aufgebrochen wird. Die Monomerflüssigkeit aus dem Monomer-Behälter 46 fließt durch das Sieb 45 beziehungsweise den Filter 45 wobei Bruchstücke des Monomer-Behälters 46 zurückgehalten werden. Durch die geneigte Bodenfläche 48 wird die Monomerflüssigkeit durch die Einmündung 44 in den Hohlzylinder 12 geleitet. Der freie Innenraum des Hohlzylinders 12 wird vollständig mit der Monomerflüssigkeit gefüllt, da in dem Monomer-Behälter 46 mehr Monomerflüssigkeit enthalten ist, als der Hohlzylinder 12 aufnehmen kann. Lufteinschlüsse entweichen durch die Einmündung 44, da diese am höchsten Punkt des durch den Hohlzylinder 12 und den Pumpkolben 14 begrenzten Raums angeordnet ist. Die Monomerflüssigkeit kann dabei nicht über den Scheitelpunkt der Schlaufe 40 fließen, da dieser deutlich oberhalb der der Einmündung 44 angeordnet ist und auch oberhalb des Flüssigkeitsspiegels der Monomerflüssigkeit im Anschluss 20 angeordnet ist, so dass die Monomerflüssigkeit daher nicht ohne Druck derart hoch steigen wird.

Der Pumpkolben 14 kann anschließend vollständig oder teilweise in den Hohlzylinder 12 eingedrückt werden, um die gewünschte Menge Monomerflüssigkeit aus dem Hohlzylinder 12 durch die Verbindungsleitung 38 in den Innenraum 24 der Kartusche 1 zu überführen beziehungsweise zu pressen. Die Menge der eingepressten Monomerflüssigkeit lässt sich durch unterschiedlich tiefes Einschieben des Pumpkolbens 14 in den Hohlzylinder 12 einstellen. Hierzu können Markierungen (nicht gezeigt) außen am Pumpkolben 14 vorgesehen sein, oder am Hohlzylinder 12 sind Markierungen angebracht, und der Hohlzylinder 12 besteht aus einem transparenten Material. Die Monomerflüssigkeit wird durch den Filter 32 in den Innenraum 24 der Kartusche 1 gepresst und steigt dort an und mischt sich mit dem Zementpulver, das in dem Innenraum 24 der Kartusche 1 gelagert ist.

Nachdem die gewünschte Menge in den Innenraum 24 der Kartusche 1 eingefüllt wurde, wird durch Hineinschieben, Herausziehen und Drehen der Mischeinrichtung 26 beziehungsweise der Mischstange 4 mit Hilfe des Griffstücks 6 manuell die Monomerflüssigkeit und das Zementpulver gemischt und so der Zementteig beziehungsweise der PMMA-Knochenzement gemischt. Nach erfolgter Durchmischung wird die Mischstange 4 bis zum Anschlag aus dem Innenraum 24 der Kartusche 1 herausgezogen und abgebrochen, damit sie später nicht stört. Gegebenenfalls kann eine Arretierung des Austragskolbens 2 gelöst werden. Anschließend wird die Kartusche 1 vom Standfuß 10 abgeschraubt und ein Austragsrohr (nicht gezeigt) kann auf das Innengewinde am Kartuschenanschluss 36 geschraubt werden. Anschließend kann der fertig gemischte Knochenzement aus dem Innenraum der Kartusche durch die Austragsöffnung und das Austragsrohr durch Einpressen des Austragskolbens 2 ausgetrieben und appliziert werden.

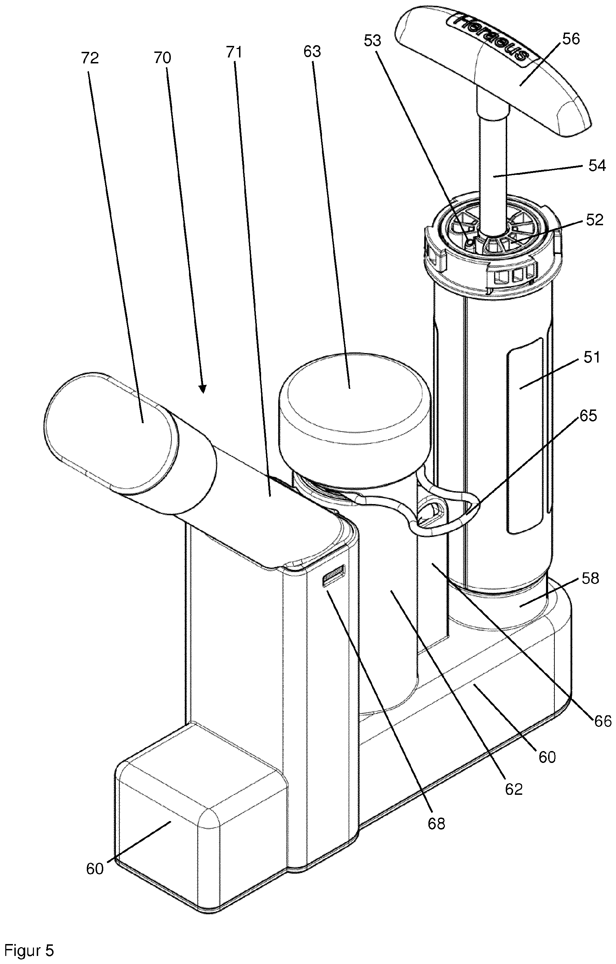

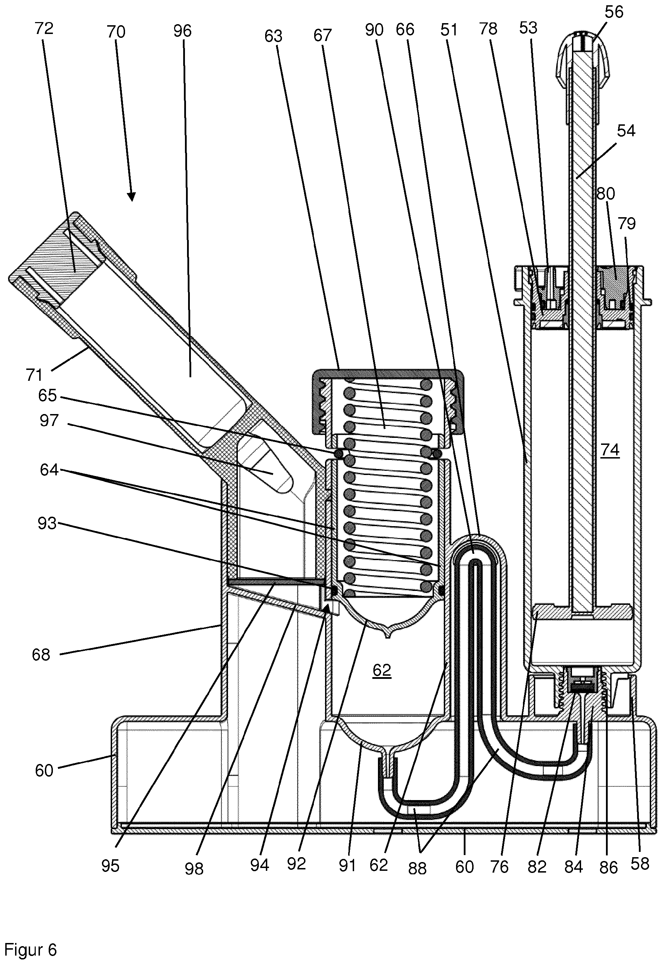

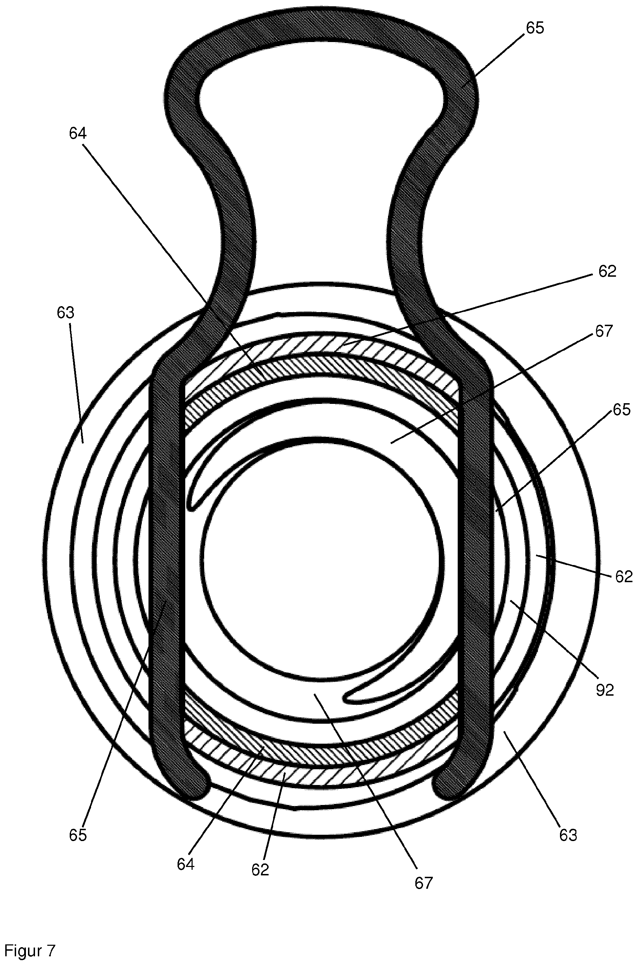

Die Figuren 5 bis 7 zeigen schematische Darstellungen einer zweiten alternativen erfindungsgemäßen Vorrichtung, die zum Durchführen eines erfindungsgemäßen Verfahrens geeignet ist. Dabei ist in Figur 5 eine schematische perspektivische Ansicht einer alternativen erfindungsgemäßen Vorrichtung als Full-Prepacked-Mischsystem dargestellt, in Figur 6 die Vorrichtung nach Figur 5 in einer schematischen Querschnittansicht und in Figur 7 eine schematische Querschnittansicht eines Teilbereichs der Vorrichtung nach den Figuren 4 und 5 dargestellt.

Die Vorrichtung umfasst eine Kartusche 51 in der ein Zementpulver als Ausgangskomponente des herzustellenden PMMA-Knochenzements enthalten ist. Auf der Oberseite (in den Figuren 5 und 6 oben) ist die Kartusche 51 mit einem Austragskolben 52 verschlossen. Der Austragskolben 52 kann mit den Wandungen der Kartusche 51 arretiert sein. In dem Austragskolben 52 ist ein Vakuumanschluss 53 vorgesehen, mit dem das Innere der Kartusche 51 evakuiert werden kann und durch den theoretisch auch Ethylenoxid zur Sterilisation des Inhalts der Kartusche 51 eingefüllt werden kann. Durch eine zentrische Durchführung in dem Austragskolben 52 ist eine Mischstange 54 geführt, die sich in die Kartusche 51 hinein und herausziehen lässt und die in dem Austragskolben 52 und damit in der Kartusche 51 drehbar gelagert ist. An dem oberen Ende der Mischstange 54 (in den Figuren 5 und 6 oben) ist ein Griffstück 56 zur manuellen Bedienung der Mischstange 4 befestigt.

Im Bereich eines Stutzens 58 an einem Standfuß 60 der Vorrichtung ist die Kartusche 51 mit dem Standfuß 60 lösbar befestigt. Einteilig mit dem Standfuß 60 ausgeführt ist an dessen Oberseite ein Hohlzylinder 62 angeordnet, der durch einen Schraubdeckel 63 verschlossen ist. In dem Hohlzylinder 62 ist ein passender Pumpkolben 64 angeordnet ist, der sich in das Innere des Hohlzylinders 62 einpressen beziehungsweise eindrücken lässt (in den Figuren 5 und 6 nach unten). Der Pumpkolben 64 ist mit Hilfe einer Spange 65 als Arretierung 65 arretiert, so dass er sich nicht ohne Lösen der Spange 65 beziehungsweise der Arretierung 65 ins Innere des Hohlzylinders 62 bewegen kann. Im Inneren des Pumpkolbens 64 ist eine gespannte Druckfeder 67 angeordnet, die sich gegen den Schraubdeckel 63 abstützt. Nach Lösen der Spange 65 beziehungsweise der Arretierung 65 durch herausziehen (in Figur 7 nach oben ziehen) drückt die Druckfeder 67 den Pumpkolben 64 nach unten in den Hohlzylinder 62 hinein.

Neben dem Hohlzylinder 62 ist am Standfuß 60 noch ein Anschlussstutzen 68 vorgesehen, der ein Teil eines Anschlusses 70 für einen Monomer-Behälter 96 der Vorrichtung bildet. Der Anschluss 70 für den Monomer-Behälter 96 umfasst neben dem Anschlussstutzen 68 noch eine elastische Aufnahme 71 für den Monomer-Behälter 96 und einen Deckel 72, mit dem der Anschluss 70 für den Monomer-Behälter 96 nach außen abschließbar ist, nachdem der Monomer-Behälter 96 einsteckt wurde. Der Monomer-Behälter 96 ist im Inneren des Anschlusses 70 und in der Vorrichtung angeordnet. Der Monomer-Behälter 96 ist eine Glasampulle, die mit einer Monomerflüssigkeit als zweiter Komponente für den herzustellenden PMMA-Knochenzement gefüllt ist. Die elastische Aufnahme 71 besteht aus Gummi oder einem anderen elastischen Kunststoff.

Weitere Details zum Aufbau der Vorrichtung sind in Figur 6 und 7 zu erkennen. Das Innere der Kartusche 51 wird durch einen zylindrischen Innenraum 74 gebildet, in dem das Zementpulver enthalten ist. Zudem ist im Innenraum 74 der Kartusche 51 eine Mischeinrichtung 76 bestehend aus mehreren Mischflügeln 76 angeordnet, die an der Mischstange 54 befestigt ist und die über die Mischstange 54 im Innenraum 74 bewegt werden kann. Der Austragskolben 52 ist zweiteilig aufgebaut und besteht aus einem Sterilisationskolben 78 (in Figur 6 oberer Teil des Austragskolbens) und einem mit einer Dichtung 79 gegen die Innenwand der Innenraums 74 abgedichteten Dichtungskolben 80 (in Figur 6 unterer Teil des Austragskolbens). Der Dichtungskolben 80 weist eine gasdurchlässige aber für Pulver undurchlässige Porenscheibe auf, durch die der Innenraum 74 evakuierbar ist. Der Austragskolben 52 hat einen zylindrischen Außenumfang und schließt dicht mit den Wänden des Innenraums 74 ab. Der Austragskolben 52 kann im Innenraum 74 in Richtung einer Austragsöffnung vorgetrieben werden, die auf der dem Austragskolben 52 gegenüberliegenden Seite des Innenraums 74 der Kartusche 51 angeordnet ist.

Zum Anschließen der Kartusche 51 an den Standfuß 60 ist ein Standfuß-Anschluss 84 mit einem Außengewinde als Verbindung zur Kartusche 1 vorgesehen, in dem ein pulverundurchlässiger und flüssigkeitsdurchlässiger Filter 82 angeordnet ist. Auf der dem Austragskolben 52 gegenüberliegenden Seite des Innenraums 74 der Kartusche 51 ist ein zum Außengewinde des Standfuß-Anschlusses 84 passender Kartuschenanschluss 86 mit Innengewinde vorgesehen. Der Kartuschenanschluss 86 begrenzt die Austragsöffnung der Kartusche 51. Der Kartuschenanschluss 86 ist auf den Standfuß-Anschluss 84 aufgeschraubt und schließt mit diesem dicht ab.

Zwischen dem Hohlzylinder 62 und dem Innenraum 74 der Kartusche 51 ist eine Verbindungsleitung 88 vorgesehen, die den Hohlzylinder 62 mit dem Innenraum 74 der Kartusche 51 verbindet. An der Einmündung in den Innenraum 74 der Kartusche 51 ist der Filter 82 angeordnet, der verhindert, dass Zementpulver aus dem Innenraum 74 der Kartusche 51 in die Verbindungsleitung 88 eindringt. Die Verbindungsleitung 88 bildet eine Schlaufe 90 mit einem hohen Scheitelpunkt, um ein unkontrolliertes Durchfließen der Monomerflüssigkeit durch die Verbindungsleitung 88 in den Innenraum 74 der Kartusche 51 zu verhindern. Ein Gehäuse 66 mit einem kleinen Sichtfenster ist zur Abdeckung und visuellen Kontrolle der Schlaufe 80 vorgesehen.

Der Stutzen 58, Standfuß 60, der Hohlzylinder 62, das Gehäuse 66 und der Anschlussstutzen 68 für den Monomer-Behälter 96 sind einteilig aus einem Kunststoff beispielsweise durch Spritzgießen hergestellt. Der Hohlzylinder 62 ist an der Unterseite (in Figur 6 unten) durch einen trichterförmigen Boden 91 begrenzt, der sich nach unten stetig verjüngt. Hierdurch wird sichergestellt, dass die Monomerflüssigkeit vollständig aus dem Hohlzylinder 62 abfließen beziehungsweise herausgedrückt werden kann. Der Pumpkolben 64 ist durch einen Hohlkörper aus einem Kunststoff gebildet und an der unteren Fläche 92 als Negativ des Bodens 91 des Hohlzylinders 62 geformt. Der Pumpkolben 64 ist mit einer Dichtung 93 gegen die Innenwand des Hohlzylinders 62 abgedichtet und in Längsrichtung (in Figur 6 nach unten) beweglich im Hohlzylinder 62 gelagert.

An einer Mantelfläche des Hohlzylinders 62, direkt unterhalb der unteren Fläche 92 des Pumpkolbens 64, ist eine Einmündung 94 von dem Anschluss 70 für den Monomer-Behälter 96 in den Hohlzylinder 62 vorgesehen. Die Einmündung 94 bildet ein Leitungsmittel für die Monomerflüssigkeit, so dass die Einmündung 94 als Teil einer Verbindungsleitung 88, 94 für die Monomerflüssigkeit aufgefasst werden kann, in der der Hohlzylinder 62 angeordnet ist.

In dem Anschluss 70 für den Monomer-Behälter 96 ist ein Sieb 95 oder Filter 95 angeordnet, mit dem Bruchstücke und Teile des geöffneten Monomer-Behälters 96 abgefangen werden können. Der Monomer-Behälter 96 ist eine Glasampulle 96 mit einem abbrechbaren Ampullenkopf 97 und einem aufbrechbaren Genick, wobei das Genick den Ampullenkopf 97 mit dem Körper der Ampulle 96 verbindet. Durch die Elastizität der Aufnahme 71 für den Monomer-Behälter 96 und durch die Verdickung der Aufnahme 71 im Bereich des Genicks kann durch Biegen der Aufnahme 71 mit dem Monomer-Behälter 96 darin der Kopf 97 des Monomer-Behälters 96 abgebrochen werden. Die Aufnahme 71 mit der entsprechenden Form, insbesondere mit der Verdickung im Bereich des Genicks, bildet dadurch ein Öffnungsmittel 71 zum Öffnen des Monomer-Behälters 96. Andere Öffnungsmittel zum Abscheren des Kopfs 97 des Monomer-Behälters 96 sind ebenfalls umsetzbar.

Im Anschlussstutzen 68 ist unterhalb des Siebs 95 beziehungsweise des Filters 95 eine geneigte Bodenfläche 98 vorgesehen, die in Richtung der Einmündung 94 geneigt ist. Dadurch kann die gesamte Monomerflüssigkeit aus dem Monomer-Behälter 96 durch die Einmündung 94 in den Hohlzylinder 62 fließen.

Ein erfindungsgemäßes Verfahren kann mit der Vorrichtung nach den Figuren 5 bis 7 beispielsweise wie folgt umgesetzt werden. Die Vorrichtung wird mit dem Standfuß 60 auf einem Tisch oder einer anderen passenden ebenen Unterlage aufgestellt. Der Monomer-Behälter 96 wird durch Knicken der elastischen Aufnahme 71 geöffnet, indem der Kopf 97 abgebrochen beziehungsweise aufgebrochen wird. Die Monomerflüssigkeit aus dem Monomer-Behälter 96 fließt durch das Sieb 95 beziehungsweise den Filter 95 wobei Bruchstücke des Monomer-Behälters 96 zurückgehalten werden. Durch die geneigte Bodenfläche 98 wird die Monomerflüssigkeit durch die Einmündung 94 in den Hohlzylinder 62 geleitet. Der freie Innenraum des Hohlzylinders 62 wird vollständig mit der Monomerflüssigkeit gefüllt, da in dem Monomer-Behälter 96 mehr Monomerflüssigkeit enthalten ist, als der Hohlzylinder 62 aufnehmen kann. Lufteinschlüsse entweichen durch die Einmündung 94, da diese am höchsten Punkt des durch den Hohlzylinder 62 und den Pumpkolben 64 begrenzten Raums angeordnet ist. Die Monomerflüssigkeit kann dabei nicht über den Scheitelpunkt der Schlaufe 90 fließen, da dieser deutlich oberhalb der der Einmündung 94 angeordnet ist und auch oberhalb des Flüssigkeitsspiegels der Monomerflüssigkeit im Anschluss 70 angeordnet ist, so dass die Monomerflüssigkeit daher nicht ohne Druck derart hoch steigen wird.

Danach wird die Spange 65 aus der Vorrichtung gezogen und damit der Pumpkolben entarretiert. Der Pumpkolben 64 wird aufgrund der meachnischen Spannung der Druckfeder 67 anschließend vollständig in den Hohlzylinder 62 eingedrückt und dadurch die Monomerflüssigkeit aus dem Hohlzylinder 62 durch die Verbindungsleitung 88 in den Innenraum 74 der Kartusche 51 überführt beziehungsweise gepresst. Die Monomerflüssigkeit wird durch den Filter 82 in den Innenraum 74 der Kartusche 51 gepresst und steigt dort an und mischt sich mit dem Zementpulver, das in dem Innenraum 74 der Kartusche 51 gelagert ist.

Nachdem die Monomerflüssigkeit in den Innenraum 74 der Kartusche 51 eingefüllt wurde, wird durch Hineinschieben, Herausziehen und Drehen der Mischeinrichtung 76 beziehungsweise der Mischstange 54 mit Hilfe des Griffstücks 56 manuell die Monomerflüssigkeit und das Zementpulver gemischt und so der Zementteig beziehungsweise der PMMA-Knochenzement gemischt. Nach erfolgter Durchmischung wird die Mischstange 54 bis zum Anschlag aus dem Innenraum 74 der Kartusche 51 herausgezogen und abgebrochen, damit sie später nicht stört. Gegebenenfalls kann eine Arretierung des Austragskolbens 52 gelöst werden. Anschließend wird die Kartusche 51 vom Standfuß 60 abgeschraubt und ein Austragsrohr (nicht gezeigt) kann auf das Innengewinde am Kartuschenanschluss 86 geschraubt werden. Anschließend kann der fertig gemischte Knochenzement aus dem Innenraum der Kartusche durch die Austragsöffnung und das Austragsrohr durch Einpressen des Austragskolbens 52 ausgetrieben und appliziert werden.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1, 51: Kartusche
- 2, 52: Austragskolben
- 3, 53: Vakuumanschluss
- 4, 54: Mischstange
- 6, 56: Griffstück
- 8, 58: Stutzen
- 10, 60: Standfuß
- 12, 62: Hohlzylinder
- 14, 64: Pumpkolben
- 16, 66: Gehäuse für Schlaufe der Verbindungsleitung
- 18, 68: Anschlussstutzen
- 20, 70: Anschluss für den Monomer-Behälter
- 21, 71: Elastische Aufnahme für den Monomer-Behälter / Öffnungsmittel
- 22, 72: Deckel
- 24, 74: Innenraum der Kartusche
- 26, 76: Mischflügel / Mischeinrichtung
- 28, 78: Sterilisationskolben
- 29, 79: Dichtung
- 30, 80: Dichtungskolben
- 32, 82: Pulverundurchlässiger und Flüssigkeitsdurchlässiger Filter
- 34, 84: Standfuß-Anschluss mit Außengewinde
- 36, 86: Kartuschenanschluss mit Innengewinde
- 38, 88: Verbindungsleitung
- 40, 90: Schlaufe der Verbindungsleitung
- 41, 91: Boden des Hohlzylinders
- 42, 92: Untere Fläche des Pumpkolbens
- 43, 93: Dichtung
- 44, 94: Einmündung in den Hohlzylinder / Verbindungsleitung
- 45, 95: Sieb / Filter
- 46, 96: Glasampulle / Monomer-Behälter
- 47, 97: Kopf der Glasampulle / Kopf des Monomer-Behälters
- 48, 98: Geneigte Bodenfläche des Anschlusses für den Monomer-Behälter
- 63: Schraubdeckel
- 65: Arretierung / Spange
- 67: Gespannte Druckfeder

## Patentansprüche

1. Vorrichtung zum Mischen von Polymethylmethacrylat-Knochenzement und zum Lagern einer Momonerflüssigkeit und eines Zementpulvers als Ausgangskomponenten des Knochenzements, die Vorrichtung aufweisend
eine Kartusche (1, 51) mit einem Innenraum (24, 74) zum Mischen des Knochenzements, der auf einer Seite durch einen beweglichen Austragskolben (2, 52) verschlossen ist,
einen Monomer-Behälter (46, 96) für eine Monomerflüssigkeit und/oder einen Anschluss (20, 70) zur Befestigung eines Monomer-Behälters (46, 96) für eine Monomerflüssigkeit, so dass der Monomer-Behälter (46, 96) in der Vorrichtung derart zu öffnen ist, dass die Monomerflüssigkeit aus dem Monomer-Behälter (46, 96) in die Vorrichtung ausläuft,
eine Verbindungsleitung (38, 88), durch die die Monomerflüssigkeit in den Innenraum (24, 74) der Kartusche (1, 51) geleitet wird,
wobei ein Hohlzylinder (12, 62) mit der Verbindungsleitung (38, 88) verbunden ist und der Hohlzylinder (12, 62) zwischen dem Monomer-Behälter (46, 96) oder dem Anschluss für den Monomer-Behälter (46, 96) und dem Innenraum (24, 74) der Kartusche (1, 51) angeordnet ist, wobei in dem Hohlzylinder (12, 62) ein axial im Hohlzylinder (12, 62) verschiebbarer Pumpkolben (14, 64) angeordnet ist, wobei die Monomerflüssigkeit aus dem geöffneten Monomer-Behälter (46, 96) in den Hohlzylinder (12, 62) fließen kann und die Verbindungsleitung (38, 88) den Hohlzylinder (12, 62) mit dem Innenraum (24, 74) der Kartusche (1, 51) derart verbindet, dass mit dem Pumpkolben (14, 64) Monomerflüssigkeit aus dem Hohlzylinder (12, 62) durch Betätigen des Pumpkolbens (14, 64) durch die Verbindungsleitung (38, 88) in den Innenraum (24, 74) der Kartusche (1, 51) gedrückt werden kann, **dadurch gekennzeichnet, dass**
der Monomer-Behälter (46, 96) für die Monomerflüssigkeit oder der Anschluss (20, 70) zur Befestigung des Monomer-Behälters (46, 96) an einer Mantelfläche des Hohlzylinders (12, 62) unmittelbar unterhalb des Pumpkolbens (14, 64) in den Hohlzylinder (12, 62) mündet.

2. Vorrichtung nach Anspruch 1, **dadurch gekennzeichnet, dass**
in der Kartusche (1, 51) eine Mischeinrichtung (26, 76) angeordnet ist, die von außen bedienbar ist, wobei vorzugsweise die Mischeinrichtung (26, 76) über eine Mischstange (4, 54) bedienbar ist, die durch eine Durchführung in dem Austragskolben (2, 52) ins Innere der Kartusche (1, 51) geführt und beweglich gelagert ist.

3. Vorrichtung nach Anspruch 1 oder 2, **dadurch gekennzeichnet, dass**
der Austragskolben (2, 52) für Pulver undurchlässig ist, wobei bevorzugt in dem Austragskolben (2, 52) ein Porenfilter angeordnet ist, der für Gas durchlässig ist und für Pulver undurchlässig ist.

4. Vorrichtung nach einem der vorangehenden Ansprüche, **dadurch gekennzeichnet, dass**
das Zementpulver im Innenraum (24, 74) der Kartusche (1, 51) enthalten ist.

5. Vorrichtung nach einem der vorangehenden Ansprüche, **dadurch gekennzeichnet, dass**
zwischen der Verbindungsleitung (38, 88) und dem Innenraum (24, 74) der Kartusche (1, 51) ein für das Zementpulver undurchlässiger und für die Monomerflüssigkeit durchlässiger Filter (32, 82) angeordnet ist.

6. Vorrichtung nach einem der vorangehenden Ansprüche, **dadurch gekennzeichnet, dass**
die Vorrichtung einen Standfuß (10, 60) aufweist, in dem zumindest ein Teil der Verbindungsleitung (38, 88) angeordnet ist, wobei die Kartusche (1, 51) lösbar mit dem Standfuß (10, 60) verbunden ist, insbesondere über ein Schraubgewinde lösbar mit dem Standfuß (10, 60) verbunden ist, wobei bevorzugt der für das Zementpulver undurchlässige und für die Monomerflüssigkeit durchlässige Filter (32, 82) in dem Standfuß (10, 60) der Vorrichtung angeordnet ist, besonders bevorzugt in der Verbindung (34, 84) zur Kartusche (1, 51) des Standfußes (10, 60) angeordnet ist.

7. Vorrichtung nach Anspruch 6, **dadurch gekennzeichnet, dass**
der Hohlzylinder (12, 62) und der Monomer-Behälter (46, 96) oder der Hohlzylinder (12, 62) und der Anschluss (20, 70) zur Befestigung des Monomer-Behälters (46, 96) mit dem Standfuß (10, 60) verbunden sind, bevorzugt unlösbar mit dem Standfuß (10, 60) verbunden sind.

8. Vorrichtung nach einem der vorangehenden Ansprüche, **dadurch gekennzeichnet, dass**
die Vorrichtung ein Öffnungsmittel (21, 71) zum Öffnen des Monomer-Behälters (46, 96) aufweist, mit dem der Monomer-Behälter (46, 96) innerhalb der Vorrichtung zu öffnen ist, wobei bevorzugt in der Verbindung zum Hohlzylinder (12, 62) ein Sieb (45, 95) oder ein Filter (45, 95) angeordnet ist, mit dem Bruchstücke oder Fetzen des geöffneten Monomer-Behälters (46, 96) zurückgehalten werden können.

9. Vorrichtung nach einem der vorangehenden Ansprüche, **dadurch gekennzeichnet, dass**
der Monomer-Behälter (46, 96) oberhalb der Verbindung zum Hohlzylinder (12, 62) angeordnet ist.

10. Vorrichtung nach einem der vorangehenden Ansprüche, **dadurch gekennzeichnet, dass**
die Verbindungsleitung (38, 44, 88, 94) an der unteren Seite mit dem Hohlzylinder (12, 62) verbunden ist, bevorzugt am tiefsten Punkt des Hohlzylinders (12, 62) mit dem Hohlzylinder (12, 62) verbunden ist, wobei besonders bevorzugt der Pumpkolben (14, 64) auf der gegenüberliegenden Seite des Hohlzylinders (12, 62) angeordnet ist.

11. Vorrichtung nach einem der vorangehenden Ansprüche, **dadurch gekennzeichnet, dass**
der Hohlzylinder (12, 62) auf der dem Pumpkolben (14, 64) gegenüberliegenden Seite einen kegelförmigen, halbkugelförmigen oder anderen sich nach unten verjüngenden Boden (41, 91) aufweist, wobei bevorzugt die dem Boden (41, 91) des Hohlzylinders (12, 62) zugewandte Fläche (42, 92) des Pumpkolbens (14, 64) eine Negativform des Bodens (41, 91) bildet.

12. Vorrichtung nach einem der vorangehenden Ansprüche, **dadurch gekennzeichnet, dass**
der Pumpkolben (14, 64) manuell axial im Hohlzylinder (12, 62) bewegt werden kann, bevorzugt manuell axial in den Hohlzylinder (12, 62) eingepresst werden kann.

13. Vorrichtung nach einem der vorangehenden Ansprüche, **dadurch gekennzeichnet, dass**
der Hohlzylinder (12, 62) transparent ist und Markierungen zur Füllstandsmenge einer Flüssigkeit in dem Hohlzylinder (12, 62) aufweist.

14. Vorrichtung nach einem der vorangehenden Ansprüche, **dadurch gekennzeichnet, dass**
der Hohlzylinder (12, 62) ein Innengewinde aufweist und der Pumpkolben (14, 64) ein dazu passendes Außengewinde aufweist, so dass der Pumpkolben (14, 64) in den Hohlzylinder (12, 62) einschraubbar ist, um die Monomerflüssigkeit aus dem Hohlzylinder (12, 62) in den Innenraum (24, 74) der Kartusche (1, 51) zu pressen.

15. Vorrichtung nach einem der Ansprüche 1 bis 12, **dadurch gekennzeichnet, dass** die Vorrichtung eine gespannte Druckfeder (67) und eine Arretierung (65) aufweist, wobei die Druckfeder (67) und/oder der Pumpkolben (64) mit der Arretierung (65) lösbar arretiert ist oder sind, wobei bei gelöster Arretierung (65) die Druckfeder (67) einen Druck auf den Pumpkolben (64) ausübt, so dass der Pumpkolben (64) in den Hohlzylinder (62) gepresst wird.

16. Vorrichtung nach einem der vorangehenden Ansprüche, **dadurch gekennzeichnet, dass**
der Austragskolben (2, 52) mit einer lösbaren Rastvorrichtung mit der Kartusche (1, 51) verbunden ist, wobei die Rastvorrichtung manuell, insbesondere durch axiale Krafteinwirkung lösbar ist, so dass der Austragskolben (2, 52) axial im Innenraum (24, 74) der Kartusche (1, 51) bewegt werden kann.

17. Vorrichtung nach einem der vorangehenden Ansprüche, **dadurch gekennzeichnet, dass**
die Verbindungsleitung (38, 44, 88, 94) zwischen dem Hohlzylinder (12, 62) und dem Innenraum (24, 74) der Kartusche (1, 51) eine nach oben weisende Schlaufe (40, 90) aufweist, wobei der höchste Punkt der Schlaufe (40, 90) oberhalb der Einmündung (44, 94) des Monomer-Behälters (46, 96) oder des Anschlusses (20, 70) für den Monomer-Behälter (46, 96) in den Hohlzylinder (12, 62) liegt.

18. Vorrichtung nach einem der vorangehenden Ansprüche, **dadurch gekennzeichnet, dass**
das Volumen im Hohlzylinder (12, 62) kleiner oder gleich dem Volumen der Monomerflüssigkeit in dem Monomer-Behälter (46, 96) ist.

19. Vorrichtung nach einem der vorangehenden Ansprüche, **dadurch gekennzeichnet, dass**
der Innenraum (24, 74) der Kartusche (1, 51) an der unteren Seite mit der Verbindungsleitung (38, 88) flüssigkeitsdurchlässig verbunden ist.

20. Verfahren zum Mischen eines Knochenzements, insbesondere mit einer Vorrichtung nach einem der Ansprüche 1 bis 19, mit den chronologischen Schritten
A) ein Monomer-Behälter (46, 96) wird geöffnet,
B) eine Monomerflüssigkeit fließt aus dem Monomer-Behälter (46, 96) in einen Hohlzylinder (12, 62), wobei der Hohlzylinder (12, 62) auf einer Seite durch einen Pumpkolben (14, 64) begrenzt ist,
C) der Pumpkolben (14, 64) wird in den Hohlzylinder (12, 62) eingedrückt und die Monomerflüssigkeit dadurch aus dem Hohlzylinder (12, 62) und durch eine Verbindungsleitung (38, 88) in den Innenraum (24, 74) einer Kartusche (1, 51) eingepresst, wobei sich in dem Innenraum (24, 74) der Kartusche (1, 51) ein Zementpulver befindet, und
D) die Monomerflüssigkeit und das Zementpulver werden in dem Innenraum (24, 74) der Kartusche (1, 51) gemischt.

21. Verfahren nach Anspruch 20, **dadurch gekennzeichnet, dass**
die Monomerflüssigkeit und das Zementpulver erst dann in dem Innenraum (24, 74) der Kartusche (1, 51) gemischt werden, wenn der Pumpkolben (14, 64) vollständig oder bis zu einer Markierung in den Hohlzylinder (12, 62) eingedrückt wurde, wobei die Markierung ein Maß für die in den Innenraum (24, 74) der Kartusche (1, 51) eingeleitete Monomerflüssigkeit ist.

22. Verfahren nach Anspruch 20 oder 21, **dadurch gekennzeichnet, dass**
die Monomerflüssigkeit und das Zementpulver in dem Innenraum (24, 74) mit einer Mischeinrichtung (26, 76) gemischt werden, indem die Mischeinrichtung (26, 76) durch Bewegen einer drehbar und in Längsrichtung verschiebbar in den Innenraum (24, 74) der Kartusche (1, 51) geführten Mischstange (4, 54) bedient wird, wobei bevorzugt nach dem Mischen die Mischstange (4, 54) bis zum Anschlag aus dem Innenraum (24, 74) der Kartusche (1, 51) herausgezogen wird und besonders bevorzugt die Mischstange (4, 54) nach dem Herausziehen bis zum Anschlag an einer Sollbruchstelle abgebrochen wird.

23. Verfahren nach einem der Ansprüche 20 bis 22, **dadurch gekennzeichnet, dass** der Monomer-Behälter (46, 96) geöffnet wird, in dem ein Öffnungsmittel (21, 71) bedient oder ausgelöst wird, wobei bevorzugt der Monomer-Behälter (46, 96) durch das Öffnungsmittel (21, 71) aufgebrochen wird.

24. Verfahren nach einem der Ansprüche 20 bis 23, **dadurch gekennzeichnet, dass** der Pumpkolben (14, 64) mit einem gespannten elastischen Federelement (67) in den Hohlzylinder (12, 62) eingedrückt wird, wobei hierfür vorzugsweise zuvor eine Arretierung (65) gelöst wird, die in den Pumpkolben (14, 64) und/oder das Federelement (67) greift.

25. Verfahren nach einem der Ansprüche 20 bis 24, **dadurch gekennzeichnet, dass** die Kartusche (1, 51) mit dem fertig gemischten Zementteig von der Verbindungsleitung (38, 44, 88, 94), dem Hohlzylinder (12, 62) und dem Monomer-Behälter (46, 96) gelöst wird und der fertig gemischte Zementteig durch Vortreiben eines Austragskolbens (2, 52), der axial beweglich in der Kartusche (1, 51) gelagert ist und der den Innenraum (24, 74) der Kartusche (1, 51) auf einer Seite begrenzt, aus dem Innenraum (24, 74) der Kartusche (1, 51) ausgetragen wird.

## Claims

1. A device for mixing of polymethylmethacrylate bone cement and for storing a monomer liquid and a cement powder as starting components of said bone cement, the device comprising
a cartridge (1, 51) with an internal space (24, 74), which is closed on one side through a mobile dispensing plunger (2, 52), for mixing the bone cement;
a monomer container (46, 96) for a monomer liquid and/or a connector (20, 70) for attaching a monomer container (46, 96) for a monomer liquid such that the monomer container (46, 96) can be opened appropriately in the device such that the monomer liquid leaks from the monomer container (46, 96) into the device;
a connecting line (38, 88) through which the monomer liquid is conducted into the internal space (24, 74) of the cartridge (1, 51);
whereby a hollow cylinder (12, 62) is connected appropriately to the connecting line (38, 88) and the hollow cylinder (12, 62) is arranged between the monomer container (46, 96) or the connector for the monomer container (46, 96) and the internal space (24, 74) of the cartridge (1, 51), whereby a pumping plunger (14, 64) that can be shifted axially in the hollow cylinder (12, 62) is arranged in the hollow cylinder (12, 62), whereby the monomer liquid can flow from the opened monomer container (46, 96) into the hollow cylinder (12, 62), and the connecting line (38, 88) appropriately connects the hollow cylinder (12, 62) to the internal space (24, 74) of the cartridge (1, 51) such that, by means of the pumping plunger (14, 64), monomer liquid can be pushed from the hollow cylinder (12, 62) through the connecting line (38, 88) into the internal space (24, 74) of the cartridge (1, 51) by actuating the pumping plunger (14, 64), **characterised in that**
the monomer container (46, 96) for the monomer liquid or the connector (20, 70) for attaching the monomer container (46, 96) merges into the hollow cylinder (12, 62) at a jacket surface of the hollow cylinder (12, 62) right below the pumping plunger (14, 64).

2. The device according to claim 1, **characterised in that**
the cartridge (1, 51) has arranged in it a mixing facility (26, 76) that can be operated from outside, whereby the mixing facility (26, 76) can preferably be operated by means of a mixing rod (4, 54) that is guided through a feedthrough in the dispensing plunger (2, 52) into the inside of the cartridge (1, 51) and is supported such as to be mobile.

3. The device according to claim 1 or 2, **characterised in that**
the dispensing plunger (2, 52) is impermeable to powder, whereby a pore filter that is permeable to gas and impermeable to powder is preferred to be arranged in the dispensing plunger (2, 52).

4. The device according to any one of the preceding claims, **characterised in that** the cement powder is contained in the internal space (24, 74) of the cartridge (1,51).

5. The device according to any one of the preceding claims, **characterised in that** a filter (32, 82) that is impermeable to the cement powder and is permeable to the monomer liquid is arranged between the connecting line (38, 88) and the internal space (24, 74) of the cartridge (1, 51).

6. The device according to any one of the preceding claims, **characterised in that** the device comprises a stand (10, 60), in which at least a part of the connecting line (38, 88) is arranged, whereby the cartridge (1, 51) is connected to the stand (10, 60) in releasable manner, in particular is connected to the stand (10, 60) in releasable manner by means of a screw thread, whereby the filter (32, 82) that is impermeable to the cement powder and is permeable to the monomer liquid is preferred to be arranged in the stand (10, 60) of the device, particularly preferably is arranged in the connection (34, 84) to the cartridge (1, 51) of the stand (10, 60).

7. The device according to claim 6, **characterised in that**
the hollow cylinder (12, 62) and the monomer container (46, 96) or the hollow cylinder (12, 62) and the connector (20, 70) for attaching the monomer container (46, 96) are connected to the stand (10, 60), preferably are connected to the stand (10, 60) in releasable manner.

8. The device according to any one of the preceding claims, **characterised in that** the device comprises an opening means (21, 71) for opening the monomer container (46, 96) by means of which the monomer container (46, 96) can be opened inside the device, whereby, preferably, a screen (45, 95) or a filter (45, 95) is arranged in the connection to the hollow cylinder (12, 62) by means of which fragments or shreds of the opened monomer container (46, 96) can be retained.

9. The device according to any one of the preceding claims, **characterised in that** the monomer container (46, 96) is arranged above the connection to the hollow cylinder (12, 62).

10. The device according to any one of the preceding claims, **characterised in that** the connecting line (38, 44, 88, 94) is connected to the hollow cylinder (12, 62) on the bottom side, preferably is connected to the hollow cylinder (12, 62) on the lowest point of the hollow cylinder (12, 62), whereby, particularly preferably, the pumping plunger (14, 64) is arranged on the opposite side of the hollow cylinder (12, 62).

11. The device according to any one of the preceding claims, **characterised in that** the hollow cylinder (12, 62) comprises, on the side opposite from the pumping plunger (14, 64), a conical, semi-spherical or otherwise downward-tapering bottom (41, 91), whereby, preferably, the surface (42, 92) of the pumping plunger (14, 64) facing the bottom (41, 91) of the hollow cylinder (12, 62) forms a negative mould of the bottom (41, 91).

12. The device according to any one of the preceding claims, **characterised in that** the pumping plunger (14, 64) can be moved axially in the hollow cylinder (12, 62) by hand, preferably can be pressed axially into the hollow cylinder (12, 62) by hand.

13. The device according to any one of the preceding claims, **characterised in that** the hollow cylinder (12, 62) is transparent and comprises markers for the filling level of a liquid in the hollow cylinder (12, 62).

14. The device according to any one of the preceding claims, **characterised in that** the hollow cylinder (12, 62) comprises an internal thread and the pumping plunger (14, 64) comprises a matching external thread such that the pumping plunger (14, 64) can be screwed into the hollow cylinder (12, 62) in order to press the monomer liquid from the hollow cylinder (12, 62) into the internal space (24, 74) of the cartridge (1, 51).

15. The device according to any one of the claims 1 to 12, **characterised in that** the device comprises a tensioned compression spring (67) and a locking mechanism (65), whereby the compression spring (67) and/or the pumping plunger (64) is or are locked in releasable manner by means of the locking mechanism (65), whereby the compression spring (67) exerts a pressure on the pumping plunger (64) when the locking mechanism (65) is released such that the pumping plunger (64) is being pressed into the hollow cylinder (62).

16. The device according to any one of the preceding claims, **characterised in that** the dispensing plunger (2, 52) is connected to the cartridge (1, 51) by means of a releasable snap-in locking device, whereby the snap-in locking device can be released manually, in particular through the action of an axial force, such that the dispensing plunger (2, 52) can be moved axially in the internal space (24, 74) of the cartridge (1, 51).

17. The device according to any one of the preceding claims, **characterised in that** the connecting line (38, 44, 88, 94) comprises an facing loop (40, 90) between the hollow cylinder (12, 62) and the internal space (24, 74) of the cartridge (1, 51), whereby the highest point of the loop (40, 90) is situated above the merging site (44, 94) into the hollow cylinder (12, 62) of the monomer container (46, 96) or of the connector (20, 70) for the monomer container (46, 96).

18. The device according to any one of the preceding claims, **characterised in that** the volume inside the hollow cylinder (12, 62) is smaller than or equal to the volume of the monomer liquid in the monomer container (46, 96).

19. The device according to any one of the preceding claims, **characterised in that** the internal space (24, 74) of the cartridge (1, 51) is connected, on the bottom side, to the connecting line (38, 88) in liquid-permeable manner.

20. A method for mixing a bone cement, in particular through the use of a device according to any one of the claims 1 to 19, comprising the chronological steps of
A) a monomer container (46, 96) being opened;
B) a monomer liquid flowing from the monomer container (46, 96) into a hollow cylinder (12, 62), whereby the hollow cylinder (12, 62) is bordered on one side by a pumping plunger (14, 64);
C) the pumping plunger (14, 64) being pushed into the hollow cylinder (12, 62) and the monomer liquid thus being pressed from the hollow cylinder (12, 62) and through a connecting line (38, 88) into the internal space (24, 74) of a cartridge (1, 51), whereby a cement powder is present in the internal space (24, 74) of the cartridge (1, 51); and
D) the monomer liquid and the cement powder being mixed in the internal space (24, 74) of the cartridge (1, 51).

21. The method according to claim 20, **characterised in that**
the monomer liquid and the cement powder are only mixed in the internal space (24, 74) of the cartridge (1, 51) once the pumping plunger (14, 64) is pushed into the hollow cylinder (12, 62) either completely or up to a marker, whereby the marker is a measure of the monomer liquid that has been conducted into the internal space (24, 74) of the cartridge (1, 51).

22. The method according to claim 20 or 21, **characterised in that**
the monomer liquid and the cement powder are mixed in the internal space (24, 74) by means of a mixing facility (26, 76) by operating the mixing facility (26, 76) by moving a mixing rod (4, 54) that is guided into the internal space 24, 74) of the cartridge (1, 51) such that it can be rotated and shifted in longitudinal direction, whereby, preferably, after the mixing, the mixing rod (4, 54) is pulled from the internal space (24, 74) of the cartridge (1, 51) up to the limit stop and, particularly preferably, the mixing rod (4, 54) is being broken off at a predetermined breakage site after being pulled out up to the limit stop.

23. The method according to any one of the claims 20 to 22, **characterised in that** the monomer container (46, 96) is being opened by operating or triggering an opening means (21, 71), whereby the monomer container (46, 96) is preferably being broken open by the opening means (21, 71).

24. The method according to any one of the claims 20 to 23, **characterised in that** the pumping plunger (14, 64) is being pushed into the hollow cylinder (12, 62) by means of a tensioned elastic spring element (67), whereby it is preferred to first release a locking mechanism (65) that engages the pumping plunger (14, 64) and/or the spring element (67).

25. The method according to any one of the claims 20 to 24, **characterised in that** the cartridge (1, 51) with the ready-mixed cement dough is being detached from the connecting line (38, 44, 88, 94), the hollow cylinder (12, 62), and the monomer container (46, 96), and the ready-mixed cement dough is being dispensed from the internal space (24, 74) of the cartridge (1, 51) by propelling a dispensing plunger (2, 52), which is supported in the cartridge (1, 51) such as to be axially mobile and which borders the internal space (24, 74) of the cartridge (1, 51) on one side.

## Revendications

1. Dispositif de mélange de ciment osseux en polyméthacrylate de méthyle et de stockage d'un fluide monomère et d'une poudre de ciment en tant que composants de départ du ciment osseux, le dispositif présentant une cartouche (1, 51) avec un espace interne (24, 74) pour le mélange du ciment osseux qui est fermé sur un côté par un piston d'extraction mobile (2, 52),
un récipient de monomère (46, 96) pour un fluide monomère et/ou un raccord (20, 70) pour la fixation d'un récipient de monomère (46, 96) pour un fluide monomère de sorte que le récipient de monomère (46, 96) est à ouvrir dans le dispositif de telle sorte que le fluide monomère s'écoule du récipient de monomère (46, 96) dans le dispositif,
une conduite de connexion (38, 88) à travers laquelle le fluide monomère est dirigé dans l'espace interne (24, 74) de la cartouche (1, 51),
dans lequel un cylindre creux (12, 62) est connecté à la conduite de connexion (38, 88) et le cylindre creux (12, 62) est disposé entre le récipient de monomère (46, 96) ou le raccord pour le récipient de monomère (46, 96) et l'espace interne (24, 74) de la cartouche (1, 51), dans lequel un piston plongeur (14, 64) pouvant être coulissé axialement dans le cylindre creux (12, 62) est disposé dans le cylindre creux (12, 62), dans lequel le fluide monomère peut s'écouler du récipient de monomère ouvert (46, 96) dans le cylindre creux (12, 62) et la conduite de connexion (38, 88) connecte le cylindre creux (12, 62) à l'espace interne (24, 74) de la cartouche (1, 51) de telle sorte que du fluide monomère peut être poussé avec le piston plongeur (14, 64) hors du cylindre creux (12, 62) par actionnement du piston plongeur (14, 64) à travers la conduite de connexion (38, 88) dans l'espace interne (24, 74) de la cartouche (1, 51), **caractérisé en ce que**
le récipient de monomère (46, 96) pour le fluide monomère ou le raccord (20, 70) pour la fixation du récipient de monomère (46, 96) à une face d'enveloppe du cylindre creux (12, 62) débouche directement en dessous du piston plongeur (14, 64) dans le cylindre creux (12, 62).

2. Dispositif selon la revendication 1, **caractérisé en ce que**
un dispositif de mélange (26, 76) qui peut être utilisé de l'extérieur est disposé dans la cartouche (1, 51), dans lequel le dispositif de mélange (26, 76) peut de préférence être utilisé par le biais d'une tige de mélange (4, 54) qui est guidée à travers une traversée dans le piston d'extraction (2, 52) dans l'intérieur de la cartouche (1, 51) et logée de manière mobile.

3. Dispositif selon la revendication 1 ou 2, **caractérisé en ce que**
le piston d'extraction (2, 52) est imperméable pour de la poudre, dans lequel un filtre à pores qui est perméable pour du gaz et est imperméable pour de la poudre est de préférence disposé dans le piston d'extraction (2, 52).

4. Dispositif selon une des revendications précédentes, **caractérisé en ce que** la poudre de ciment est contenue dans l'espace interne (24, 74) de la cartouche (1, 51).

5. Dispositif selon une des revendications précédentes, **caractérisé en ce que** un filtre (32, 82) imperméable pour la poudre de ciment et perméable pour le fluide monomère est disposé entre la conduite de connexion (38, 88) et l'espace interne (24, 74) de la cartouche (1, 51).

6. Dispositif selon une des revendications précédentes, **caractérisé en ce que** le dispositif présente un pied (10, 60) dans lequel au moins une partie de la conduite de connexion (38, 88) est disposée, dans lequel la cartouche (1, 51) est connectée au pied (10, 60) de manière amovible, est notamment connectée au pied (10, 60) de manière amovible par le biais d'un filetage vissé, dans lequel le filtre (32, 82) imperméable pour la poudre de ciment et perméable pour le fluide monomère est de préférence disposé dans le pied (10, 60) du dispositif, est disposé de manière particulièrement préférée dans la connexion (34, 84) à la cartouche (1, 51) du pied (10, 60).

7. Dispositif selon la revendication 6, **caractérisé en ce que**
le cylindre creux (12, 62) et le récipient de monomère (46, 96) ou le cylindre creux (12, 62) et le raccord (20, 70) pour la fixation du récipient de monomère (46, 96) sont connectés au pied (10, 60), sont de préférence connectés au pied (10, 60) de manière indissociable.

8. Dispositif selon une des revendications précédentes, **caractérisé en ce que** le dispositif présente un moyen d'ouverture (21, 71) pour l'ouverture du récipient de monomère (46, 96) avec lequel le récipient de monomère (46, 96) est à ouvrir au sein du dispositif, dans lequel un tamis (45, 95) ou un filtre (45, 95) avec lequel des fragments ou bribes du récipient de monomère ouvert (46, 96) peuvent être retenus est de préférence disposé dans la connexion au cylindre creux (12, 62).

9. Dispositif selon une des revendications précédentes, **caractérisé en ce que** le récipient de monomère (46, 96) est disposé au-dessus de la connexion au cylindre creux (12, 62).

10. Dispositif selon une des revendications précédentes, **caractérisé en ce que** la conduite de connexion (38, 44, 88, 94) est connectée au cylindre creux (12, 62) sur le côté inférieur, est de préférence connectée au cylindre creux (12, 62) au point le plus profond du cylindre creux (12, 62), dans lequel le piston plongeur (14, 64) est disposé de manière particulièrement préférée sur le côté opposé du cylindre creux (12, 62).

11. Dispositif selon une des revendications précédentes, **caractérisé en ce que** le cylindre creux (12, 62) présente sur le côté opposé au piston plongeur (14, 64) un fond (41, 91) conique, hémisphérique ou autre rétrécissant vers le bas, dans lequel la face (42, 92) du piston plongeur (14, 64) tournée vers le fond (41, 91) du cylindre creux (12, 62) forme un moule négatif du fond (41, 91).

12. Dispositif selon une des revendications précédentes, **caractérisé en ce que** le piston plongeur (14, 64) peut être déplacé axialement dans le cylindre creux (12, 62) manuellement, peut de préférence être enfoncé axialement dans le cylindre creux (12, 62) manuellement.

13. Dispositif selon une des revendications précédentes, **caractérisé en ce que** le cylindre creux (12, 62) est transparent et présente des marquages pour la quantité de niveau d'un fluide dans le cylindre creux (12, 62).

14. Dispositif selon une des revendications précédentes, **caractérisé en ce que** le cylindre creux (12, 62) présente un filetage interne et le piston plongeur (14, 64) présente un filetage externe adapté à celui-ci de sorte que le piston plongeur (14, 64) peut être vissé dans le cylindre creux (12, 62) pour presser le fluide monomère hors du cylindre creux (12, 62) dans l'espace interne (24, 74) de la cartouche (1, 51).

15. Dispositif selon une des revendications 1 à 12, **caractérisé en ce que** le dispositif présente un ressort de compression tendu (67) et un dispositif de blocage (65), dans lequel le ressort de compression (67) et/ou le piston plongeur (64) est ou sont bloqué(s) avec le dispositif de blocage (65) de manière amovible, dans lequel en cas de dispositif de blocage débloqué (65), le ressort de compression (67) exerce une pression sur le piston plongeur (64) de sorte que le piston plongeur (64) est pressé dans le cylindre creux (62).

16. Dispositif selon une des revendications précédentes, **caractérisé en ce que** le piston d'extraction (2, 52) est connecté à la cartouche (1, 51) avec un dispositif d'encliquetage amovible, dans lequel le dispositif d'encliquetage peut être détaché manuellement, notamment par action de force axiale de sorte que le piston d'extraction (2, 52) peut être déplacé axialement dans l'espace interne (24, 74) de la cartouche (1, 51).

17. Dispositif selon une des revendications précédentes, **caractérisé en ce que** la conduite de connexion (38, 44, 88, 94) présente entre le cylindre creux (12, 62) et l'espace interne (24, 74) de la cartouche (1, 51) une boucle tournée vers le haut (40, 90), dans lequel le point le plus haut de la boucle (40, 90) se situe au-dessus de l'embouchure (44, 94) du récipient de monomère (46, 96) ou du raccord (20, 70) pour le récipient de monomère (46, 96) dans le cylindre creux (12, 62).

18. Dispositif selon une des revendications précédentes, **caractérisé en ce que** le volume dans le cylindre creux (12, 62) est inférieur ou égal au volume du fluide monomère dans le récipient de monomère (46, 96).

19. Dispositif selon une des revendications précédentes, **caractérisé en ce que** l'espace interne (24, 74) de la cartouche (1, 51) est connecté sur le côté inférieur à la conduite de connexion (38, 88) de manière perméable au fluide.

20. Procédé de mélange d'un ciment osseux, notamment avec un dispositif selon une des revendications 1 à 19, avec les étapes chronologiques
A) un récipient de monomère (46, 96) est ouvert,
B) un fluide monomère s'écoule hors du récipient de monomère (46, 96) dans un cylindre creux (12, 62), dans lequel le cylindre creux (12, 62) est limité sur un côté par un piston plongeur (14, 64),
C) le piston plongeur (14, 64) est enfoncé dans le cylindre creux (12, 62) et le fluide monomère est enfoncé de ce fait hors du cylindre creux (12, 62) et à travers une conduite de connexion (38, 88) dans l'espace interne (24, 74) d'une cartouche (1, 51), dans lequel une poudre de ciment se trouve dans l'espace interne (24, 74) de la cartouche (1, 51), et
D) le fluide monomère et la poudre de ciment sont mélangés dans l'espace interne (24, 74) de la cartouche (1, 51).

21. Procédé selon la revendication 20, **caractérisé en ce que** le fluide monomère et la poudre de ciment ne sont mélangés dans l'espace interne (24, 74) de la cartouche (1, 51) que lorsque le piston plongeur (14, 64) a été enfoncé entièrement ou jusqu'à un marquage dans le cylindre creux (12, 62), dans lequel le marquage est une mesure pour le fluide monomère introduit dans l'espace interne (24, 74) de la cartouche (1, 51).

22. Procédé selon la revendication 20 ou 21, **caractérisé en ce que** le fluide monomère et la poudre de ciment sont mélangés dans l'espace interne (24, 74) avec un dispositif de mélange (26, 76) **en ce que** le dispositif de mélange (26, 76) est utilisé par déplacement d'une tige de mélange (4, 54) guidée à rotation et de manière coulissante dans la direction longitudinale dans l'espace interne (24, 74) de la cartouche (1, 51), dans lequel la tige de mélange (4, 54) est de préférence sortie de l'espace interne (24, 74) de la cartouche (1, 51) jusqu'à la butée après le mélange et la tige de mélange (4, 54) est cassée de manière particulièrement préférée au niveau d'un point destiné à la rupture après la sortie jusqu'à la butée.

23. Procédé selon une des revendications 20 à 22, **caractérisé en ce que** le récipient de monomère (46, 96) est ouvert **en ce qu'**un moyen d'ouverture (21, 71) est utilisé ou déclenché, dans lequel le récipient de monomère (46, 96) est de préférence perforé par le moyen d'ouverture (21, 71).

24. Procédé selon une des revendications 20 à 23, **caractérisé en ce que** le piston plongeur (14, 64) est enfoncé dans le cylindre creux (12, 62) avec un élément de ressort élastique tendu (67), dans lequel un dispositif de blocage (65) est de préférence débloqué à cet effet au préalable, lequel s'engrène dans le piston plongeur (14, 64) et/ou l'élément de ressort (67).

25. Procédé selon une des revendications 20 à 24, **caractérisé en ce que** la cartouche (1, 51) avec la pâte de ciment mélangée prête est détachée de la conduite de connexion (38, 44, 88, 94), du cylindre creux (12, 62) et du récipient de monomère (46, 96), et la pâte de ciment mélangée prête est extraite de l'espace interne (24, 74) de la cartouche (1, 51) par poussée d'un piston d'extraction (2, 52) qui est logé axialement de manière mobile dans la cartouche (1, 51) et qui limite l'espace interne (24, 74) de la cartouche (1, 51) sur un côté.
